# FASCICULE DE BREVET EUROPEEN

(11) **EP 2 430 027 B1**
(45) Date de publication et mention de la délivrance du brevet: **10.02.2016**
(21) Numéro de dépôt: 10722996.5
(22) Date de dépôt: 17.05.2010
(51) Int. Cl.: C07D 493/00, C07D 493/04, C07D 519/00, C08G 73/00, C09D 179/06

(54) **POLYMERES TRIAZOLES/TETRAZOLES ISSUS DE LA CYCLO ADDITION DE MONOMERES DERIVES DE DIANHYDROHEXITOL FONCTIONNALISES, COMPOSES INTERMEDIAIRES, LEURS PROCEDES DE PREPARATION ET LEURS APPLICATIONS**
TRIAZOL-/TETRAZOLPOLYMERE AUS DER CYCLOADDITION VON FUNKTIONALISIERTEN MONOMEREN AUS DIANHYDROHEXITOL, ZWISCHENVERBINDUNGEN, VERFAHREN ZUR HERSTELLUNG UND ANWENDUNG
TRIAZOLE/TETRAZOLE POLYMERS RESULTING FROM THE CYCLOADDITION OF FUNCTIONALIZED MONOMERS DERIVED FROM DIANHYDROHEXITOL, INTERMEDIATE COMPOUNDS, METHODS OF PREPARING SAME AND APPLICATIONS THEREOF

(30) Priorité: 15.05.2009 FR 0953256
(43) Date de publication de la demande: 21.03.2012
(73) Titulaire: Roquette Frères, 62136 Lestrem (FR)
(72) Inventeur: FUERTES, Patrick, F-59160 Lomme (FR); IBERT, Mathias, F-59930 La Chapelle d'Armentieres (FR); FLEURY, Etienne, F-69540 Irigny (FR); BERNARD, Julien, F-69001 Lyon (FR); DROCKENMULLER, Eric, F-69003 Lyon (FR); BESSET, Céline, F-69100 Villeurbanne (FR)
(74) Mandataire: Cabinet Plasseraud
(86) Numéro de dépôt international: PCT/EP2010/056736
(87) Numéro de publication internationale: WO 2010/130840

(56) Documents cités:
- EP-A1- 0 747 382
- EP-A1- 0 773 250
- WO-A2-2007/121883
- DE-A1- 19 717 371
- JP-A- 2001 316 668

## Description

### 1. Domaine de l'invention

La présente invention est relative à de nouveaux polymères (ou oligomères) obtenus à partir de monomères dérivés de 1,4 :3,6-dianhydrohexitol, à certains de ces monomères nouveaux. Des composés intermédiaires (dimères) sont aussi décrits. Ces polymères sont obtenus à partir de ces monomères selon le mécanisme chimique désigné par cycloaddition 1,3 dipolaire entre un azoture et un alcyne communément appelée "*click chemistry*", dans lequel un motif réactif azoture réagit avec un motif réactif du type alcyne ou nitrile, pour former un motif de type triazole ou tétrazole.

L'invention concerne également les procédés d'obtention de ces composés ainsi que leurs applications dans des procédés de mise en forme de matériaux polymères, par exemple des procédés d'extrusion, d'extrusion-soufflage, de moulage par injection, de moulage par compression, ou dans des procédés de revêtement, etc.

### Arrière-plan technologique et art antérieur

Le développement de matériaux polymères issus de ressources biologiques renouvelables est devenu un impératif écologique et économique, face à l'épuisement et à la montée des prix des ressources fossiles, comme le pétrole.
Dans ce contexte, la valorisation des 1,4 :3,6-dianhydrohexitols comme monomères dans des réactions de polymérisation a été envisagée. Ces monomères sont obtenus par transformation des (poly)saccharides extraits de végétaux et les polymères qui en sont issus présentent l'avantage d'être potentiellement biodégradables.
Les 1,4 :3,6-dianhydrohexitols connus sont notamment : l'isosorbide, l'isomannide et l'isoidide de formule :

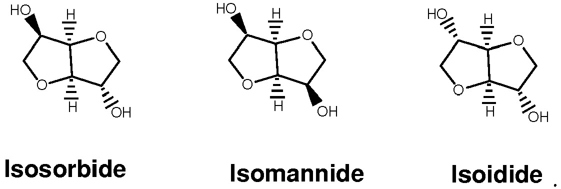

On connaît également des dérivés de l'isosorbide, l'isomannide et l'isoidide dans lesquels les fonctions réactives -OH sont remplacées par des fonctions réactives amine ou acide. Ces fonctions réactives hydroxyle, amine ou acide peuvent éventuellement être protégées par des groupements appropriés.

Les demandes de brevet FR-A-2.478.105 et WO-A-2007/121883 décrivent des dérivés mono et diazido de 1,4 :3,6-dianhydrohexitols, tels que :

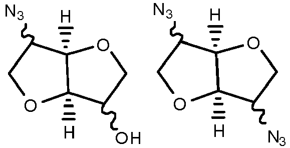

Selon ces demandes de brevet, ces dérivés sont utilisés comme principes actifs pharmaceutiques. Aucune application comme monomère dans des réactions de polymérisation, n'est divulguée.

Pour ce qui est des polymères dérivés de 1,4:3,6-dianhydrohexitol, on connait essentiellement :
- les polyesters aliphatiques ou semi-aromatiques obtenus par estérification de 1,4 :3,6-dianhydrohexitol avec des chlorures d'acide (réaction de Shotten-Bauman),
- les polyamides obtenus par réaction entre un dérivé diamino-1,4:3,6-dianhydrohexitol et divers chlorure d'acide,
- les polycarbonates obtenus soit par transestérification du diphénylcarbonate, du phosgène ou du diphosgène avec le 1,4 :3,6-dianhydrohexitol, comme suit : 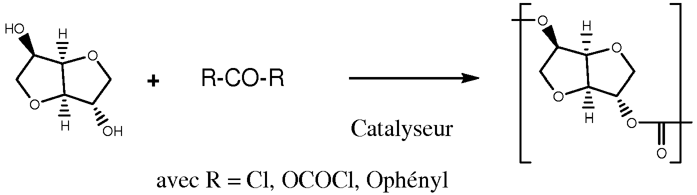 soit par polycondensation en milieu solvant du dérivé bischloroformate du 1,4 :3,6-dianhydrohexitol, comme ci-dessous : 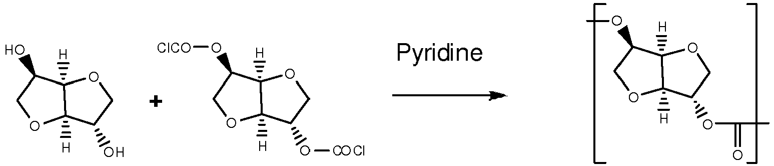
- les polyuréthanes et polyurées, tels que : 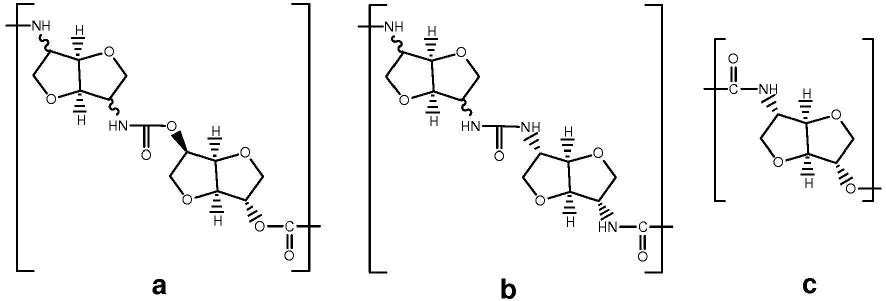
- les polyéthers obtenus directement par réaction entre le 1,4 :3,6-dianhydrohexitol et un dérivé halogéné.

On connaît par ailleurs le mécanisme de la réaction de Huisgen. Huisgen et Szeimies [(a) Huisgen, R.; Szeimies, G.; Moebius, L. Chem. Ber. 1967, 100, 2494. (b) Huisgen, R.; Knorr, R.; Moebius, L.; Szeimies, G. Chem. Ber. 1965, 98, 4014] ont, les premiers, réalisé la cycloaddition 1,3-dipolaire non catalysée d'un dérivé azido sur un dérivé alcyne à haute température qui donne un mélange de composé 1,4 et 1,5 disubstitués. Le schéma de cette cycloaddition est le suivant :

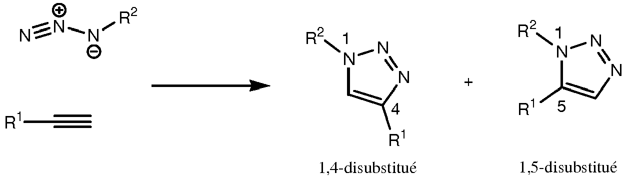

La demande de brevet WO-A-03/101972 décrit la réaction de cycloaddition 1,3 dipolaire (dite de "Huisgen"), entre des azotures et des alcynes, en présence d'un catalyseur de cuivre I. Cette réaction permet de former, de manière régiospécifique, essentiellement du 1,2,3-triazole 1-4-disubstitué. Comme montré dans les figures 3A et 3B du WO-A-03/101972, cette cycloaddition 1,3 dipolaire permet d'obtenir, par exemple, des systèmes hybrides (cf. produits 1 à 10) comprenant d'une part, des noyaux phényles et, d'autre part, des molécules cycliques inertes ou ramifiés, éventuellement insaturées et éventuellement porteuses d'hydroxyles ainsi qu'un système hybride (11) comprenant une rotule triazole reliant, d'une part, un reste propane diol et, d'autre part, un composé polycyclique dihydroxylé. La demande de brevet WO-A-03/101972 ne divulgue pas de composés dérivés de 1,4 :3,6-dianhydrohexitol.
D'après la demande WO 2007/041451 A, en présence d'un catalyseur de ruthénium II, les composés 1,5 disubstitués sont privilégiés. (Rasmussen et al. Org. Lett, 9 (26), 5337-5339, 2007 et Sharpless et al., J. Am. Chem. Soc., 127 (46), 15998-15999, 2005).

La demande de brevet WO-A-2007/121883 décrit des composés issus de la réaction de *click chemistry* entre un composé 2,5-diazido-1,4 :3,6-dianhydrohexitol et des nitriles ou des alcynes aromatiques. Aucun des composés décrits dans cette demande de brevet n'est un polymère.

La demande PCT WO-A-2008/048733 décrit un procédé de polymérisation en masse par réaction de cycloaddition 1,3-dipolaire en présence de cuivre. Les espèces réactives mises en oeuvre ne sont pas des dérivés de 1,4 :3,6-dianhydrohexitol. Ces espèces réactives comprennent des monomères à fonction alcyne mais également des oligomères, des polymères à fonction alcyne tels que :

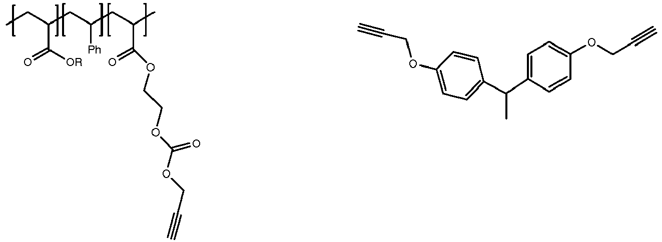

Force est donc de constater que l'art antérieur ne divulgue pas de monomères de type 1,4:3,6-dianhydrohexitol utilisés pour la synthèse de dimères, d'oligomères et de polymères à motifs triazole ou tétrazole par "*click chemistry*".

### Objectifs

Dans ce contexte, l'un des objectifs essentiels de la présente invention est de fournir de nouveaux polymères obtenus à partir de monomères dérivés de 1,4 :3,6-dianhydrohexitol issus d'amidons.
Un autre objectif essentiel de l'invention est de fournir de nouveaux monomères dérivés de 1,4 :3,6-dianhydrohexitol susceptibles de réagir par réaction de "*click chemistry*", seuls ou en présence d'autres monomères et présentant tout ou partie des caractéristiques suivantes :
- multifonctionnels,
- polychiraux,
- de stéréochimie contrôlée,
- et susceptibles d'être obtenus par un procédé simple et économique.
Un autre objectif essentiel de l'invention est de fournir des polymères à motif triazole ou tétrazole qui soient riches en motifs 1,4 :3,6-dianhydrohexitol, et qui présentent tout ou partie des caractéristiques suivantes :
- une température de transition vitreuse élevée,
- susceptibles d'être mis en forme par les techniques classiques de transformation des plastiques notamment extrusion, extrusion-soufflage, ou moulage par injection ou par compression ou par coating,
- des qualités mécaniques,
- économiques.

Sont également décrits des composés intermédiaires (dimères ou oligomères à motif triazole ou tétrazole et 1,4 :3,6-dianhydrohexitol) qui soient réactifs et qui permettent d'obtenir aisément de nombreux types de polymères par des réactions de polymérisation ultérieures.
Un autre objectif essentiel de l'invention est de fournir des polymères dont le degré de polymérisation est aisément contrôlé et qui permettent d'obtenir ultérieurement des polymères à plus haut degré de polymérisation, riches en motifs 1,4 :3,6-dianhydrohexitol, par simple réactivation thermique.
Encore un autre objectif de l'invention est de fournir un procédé de préparation, à partir des monomères dérivés de 1,4 :3,6-dianhydrohexitol multifonctionnels, de polymères à motif triazole ou tétrazole riches en motifs 1,4 :3,6-dianhydrohexitol, et présentant tout ou partie des caractéristiques suivantes :
- simple,
- sans étapes lourdes de protection/déprotection des fonctions réactives,
- régiosélectif,
- économique,
- rapide,
- efficace dans des conditions douces de température et/ou rapides, en présence ou non de solvant et de catalyseur.

### Brève description de l'invention

Ces objectifs, parmi d'autres, sont atteints par la présente invention qui concerne tout d'abord des polymères obtenus à partir :
* de monomère(s) dérivé(s) de 1,4 : 3,6-dianhydrohexitol de formule I 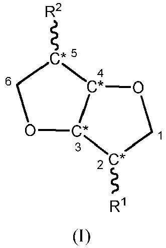 dans laquelle :
   - R¹ est un radical : 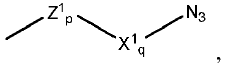 -Z²R³, ou
      -Z¹H ;
      avec p = 0 ou 1 et q = 0 ou 1 ;
   - R² est un radical -Z²R³, les radicaux -Z²R³ étant identiques ou différents quand R¹ et R² correspondent chacun à -Z²R³ ;
   - Z¹, Z² représentent indépendamment un radical divalent correspondant à : 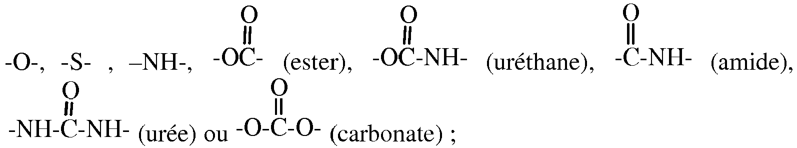
   - R³ est un radical divalent de formule : -X²-C≡Y ;
   - -X¹-, X²- sont indépendamment des liaisons covalentes ou des rotules divalentes formées par un groupement (cyclo)aliphatique et/ou aromatique, halogénés ou non et contenant ou non des hétéroatomes, de préférence un groupement -(CH₂)ₙ- avec n compris entre 1 et 100 ;
   - Y représente CH ou N ;
      lesdits polymères étant
   - soit des polymères de type AB caractérisés en ce qu'ils répondent à la formule VI suivante : 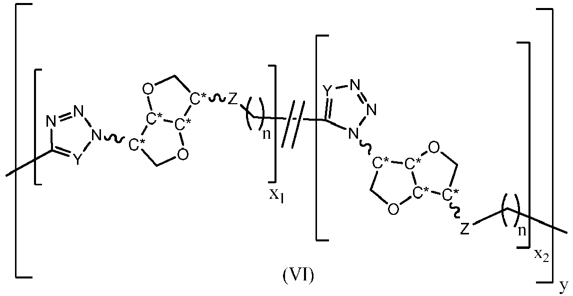 dans laquelle
      - y est le degré de polymérisation moyen,
      - x₁ et x₂ varient de 0 à 100% en fonction de la présence (ou non) et de la nature du catalyseur,
      - n est compris entre 1 et 100,
      - Y est CH ou N,
      - Z est O, S ou NH ;
   - soit des polymères de type AA/BB caractérisés en ce qu'ils répondent à la formule VII suivante : 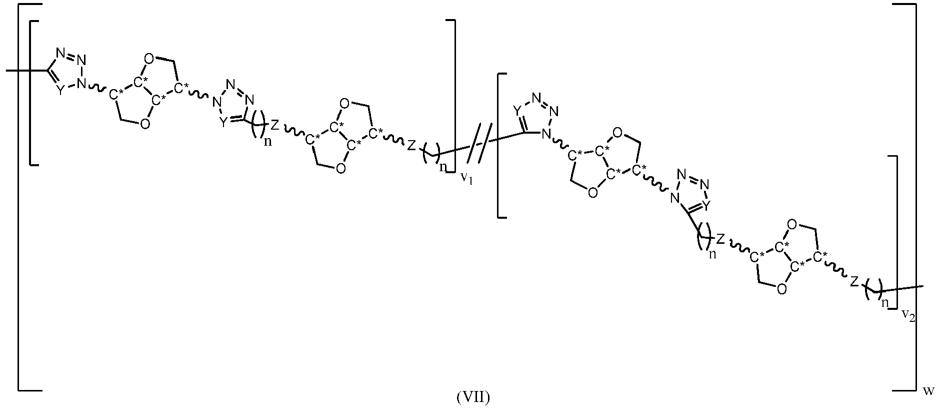
   dans laquelle
   - w est le degré de polymérisation moyen,
   - v₁ et v₂ varient de 0 à 100% en fonction de la présence (ou non) et de la nature du catalyseur,
   - n est compris entre 1 et 100,
   - Y est CH ou N,
   - Z est O, S ou NH.
Un autre objet de l'invention est relatif aux procédés de préparation de ces polymères.

L'invention se rapporte aussi à un procédé de mise en forme des nouveaux polymères selon l'invention, avec éventuellement une polymérisation des monomères de l'invention au cours de la mise en forme, et à un procédé de revêtement sur tous supports des nouveaux polymères selon l'invention.

Un autre objet de l'invention est un monomère dérivé de 1,4 : 3,6-dianhydrohexitol de formule Ia : 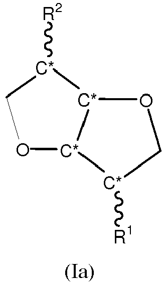 dans laquelle :
- R¹ est un radical : 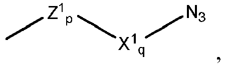
   - Z²R³, ou
   - Z¹H;
      avec p = 0 ou 1 et q = 0 ou 1 ;
- R² est un radical -Z²R³, les radicaux -Z²R³ étant identiques ou différents quand R¹ et R² correspondent chacun à -Z²R³;
- Z¹, Z² représentent indépendamment un radical divalent correspondant à : 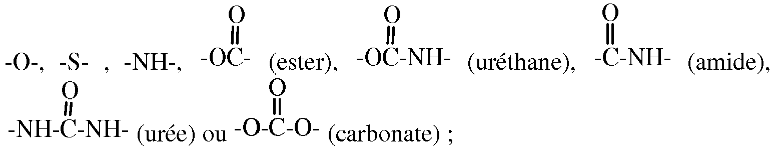
- R³ est un radical divalent de formule : -X²-C≡Y ;
- -X¹-, -X²- sont indépendamment un groupement -(CH₂)ₙ- avec n compris entre 1 et 100 ;
- Y représente CH.

Dans cette formule, les carbones C2*, C3*, C4* et C5* sont des carbones chiraux ; les carbones C3* et C4* étant de préférence de configuration exo (S).
Dans cette formule et dans toutes les formules données dans le présent exposé, des groupements (cyclo)aliphatiques et/ou aromatiques, halogénés ou non et comportant ou non des hétéroatomes, peuvent se substituer à tout ou partie des atomes d'hydrogène substituables.
Ces nouveaux monomères sont faciles à préparer avec un coût de revient acceptable. Ils sont donc parfaitement adaptés à une exploitation industrielle et ils ouvrent la porte à de nombreuses utilisations, en particulier la synthèse d'oligomères ou de polymères. Ces polymères sont des matières plastiques transformables, notamment par extrusion, extrusion-soufflage, ou moulage par injection ou par compression. Des applications de ces polymères dans des procédés de revêtement sont également envisageables.
La présente description propose également un procédé de préparation d'un monomère formule I' suivante : 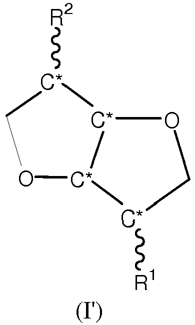 dans laquelle
- R¹ et R² sont identiques ou différents entre eux et choisis parmi les radicaux : 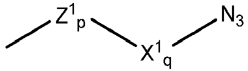 et -Z²R³ ; R¹ pouvant également correspondre à -Z¹H quand R² correspond à -Z²R³ ;
- Z¹, Z² représentent indépendamment un radical divalent correspondant à : 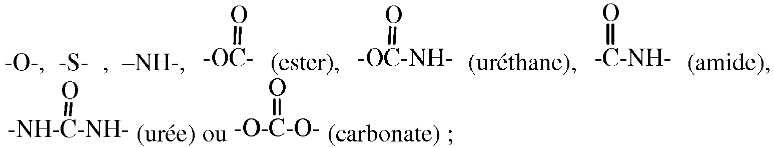
- R³ est un radical divalent de formule : -X²-C≡Y ;
- -X¹-, -X²- sont indépendamment une liaison covalente ou des rotules divalentes formées par un groupement (cyclo)aliphatique et/ou aromatique, halogéné ou non et contenant ou non des hétéroatomes, de préférence un groupement : -(CH₂)ₙ- avec n compris entre 1 et 100 ; et
- Y représente CH ou N ;
caractérisé en ce que l'on fait réagir un dérivé 1,4:3,6-dianhydrohexitol de formule II : 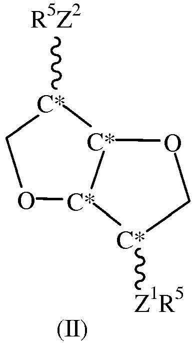 dans laquelle les radicaux R⁵ sont identiques ou différents et représentent un hydrogène ou un alkyle en C1-C10; -Z¹R⁵ et -Z²R⁵ pouvant être par exemple -OH; -NH₂; -SH; -OCH₃ ; avec au moins un précurseur des radicaux R¹ et/ou R².

L'homme de l'art est à même de sélectionner les précurseurs des radicaux R¹ et/ou R² et de les mettre en oeuvre dans des réactions chimiques classiques, qui conduiront aux différents types de monomères de formule I'.

Quand de manière préférée, -Z¹R⁵ et -Z²R⁵ correspondent chacun à -OH, alors les précurseurs des radicaux R¹ et/ou R² peuvent être :
- MN₃ ; M correspondant à H ou à un métal, de préférence Na
   ou
- -Z²R³, OCNR³ ou R⁴Z²R³; avec R⁴ correspondant à H ou un halogène.

Un tel procédé est particulièrement avantageux par sa simplicité, son économie, son éco-compatibilité et la multiplicité des configurations qu'il permet de conférer aux monomères visés.

Sont également décrits des composés intermédiaires (dimères) de formules III et III' : 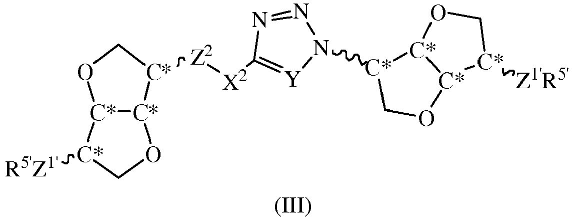 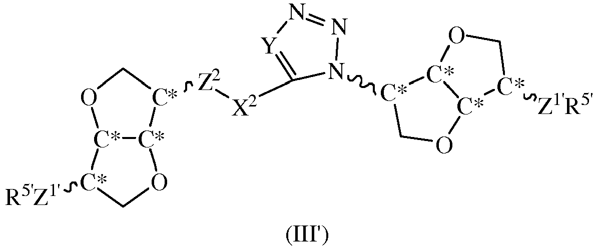 dans lesquelles
- YestCHouN;
- Z² représente un radical divalent correspondant à : -O-, -S-, -NH-, 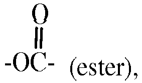 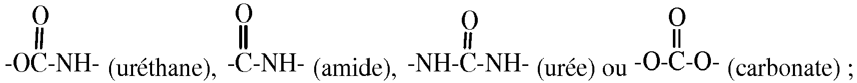
- -X²- est une liaison covalente ou une rotule divalente formée par un groupement (cyclo)aliphatique et/ou aromatique, halogéné ou non et contenant ou non des hétéroatomes, de préférence un groupement : -(CH₂)ₙ- avec n compris entre 1 et 100 ;
- Z^{1'} représente radical divalent correspondant à : -O-, -S-, -NH-, 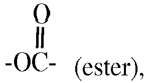 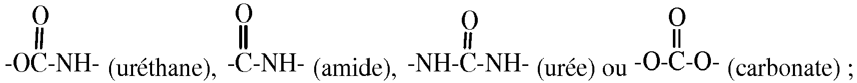
- R^{5'} représente un hydrogène, un alkyle ou un aryle en C1-C10; -Z^{1'}R^{5'} pouvant être par exemple -OH; -NH₂; -SH; -OCH₃.
Les carbones C* sont des carbones chiraux.

Un procédé de préparation du composé intermédiaire tel que défini est également décrit. Ce procédé comprend la réaction entre un dérivé de 1,4:3,6-dianhydrohexitol de formule IV : 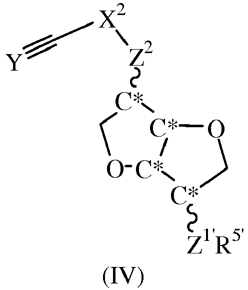 dans laquelle
- Z² représente un radical divalent correspondant à : 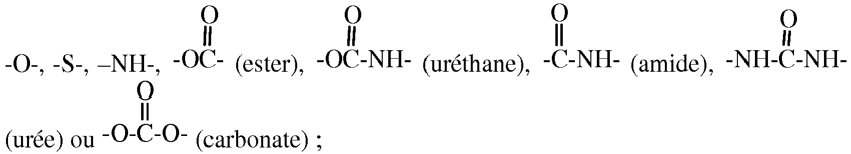
- -X²- est une liaison covalente ou une rotule divalente formée par un groupement (cyclo)aliphatique et/ou aromatique, halogéné ou non et contenant ou non des hétéroatomes, de préférence un groupement : -(CH₂)ₙ- avec n compris entre 1 et 100 ;
- Z^{1'} représente radical divalent correspondant à : 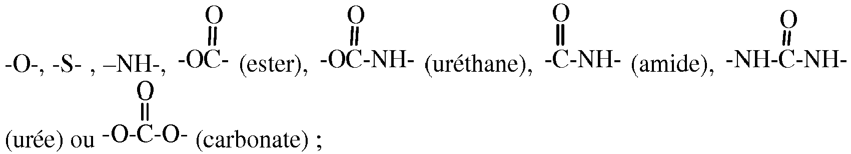
- R^{5'} représentant un hydrogène ou un alkyle en C1-C10; -Z^{1'}R^{5'} pouvant être par exemple -OH; -NH₂; -SH; -OCH₃ ;
- YestCHouN;
   avec un composé de formule V : 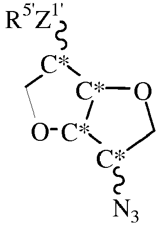
dans laquelle :
- Z^{1'} représente radical divalent correspondant à : 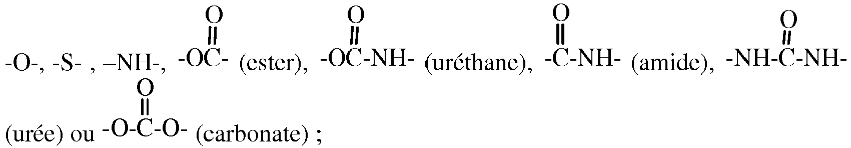
- R^{5'} représentant un hydrogène ou un alkyle ou un aryle en C1-C10; -Z^{1'}R^{5'} pouvant être par exemple -OH; -NH₂; -SH; -OCH₃.

Dans ces deux formules IV et V les carbones C2*, C3*, C4* et C5* sont des carbones chiraux ; les carbones C3* et C4* étant de préférence de configuration exo (S).

De manière générale dans tout le présent exposé, les carbones C* sont des carbones chiraux.

### Description détaillée de l'invention

### Les Monomères

Les monomères de formule I ou I', peuvent être subdivisés en plusieurs types dénommés arbitrairement "AA", "BB" et "AB" dans le présent exposé, selon la nature des radicaux R¹ et R².

### Monomères "AA"

Ces monomères selon l'invention sont des monomères de formule I ou I' dans laquelle R¹ et R² sont identiques et représentent un radical -Z²R³ avec :
- Z² représentant un radical divalent correspondant à : 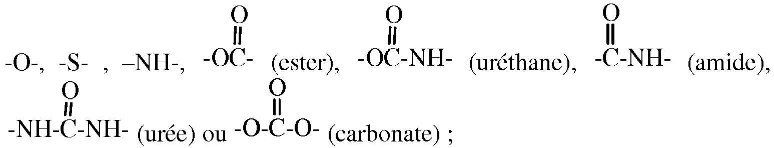
- R³ étant un radical divalent de formule : -X²-C≡Y ;
- -X²- étant une liaison covalente ou une rotule divalente formée par un groupement (cyclo)aliphatique et/ou aromatique, halogéné ou non et contenant ou non des hétéroatomes, de préférence un groupement -(CH₂)ₙ-avec n compris entre 1 et 100 ;
- Y représentant CH ou N.

Il s'agit notamment de monomères répondant à la formule développée I-1 ci-dessous : 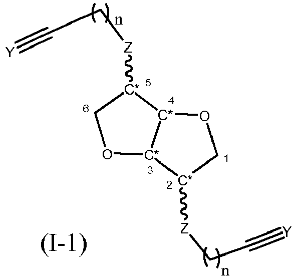 dans laquelle :
- Z est O, S ou NH,
- n compris entre 1 et 100, et
- Y est CH ou N.

De préférence, les monomères de type "AA" de l'invention répondent aux formules développées I-1a à I-1c ci-dessous : 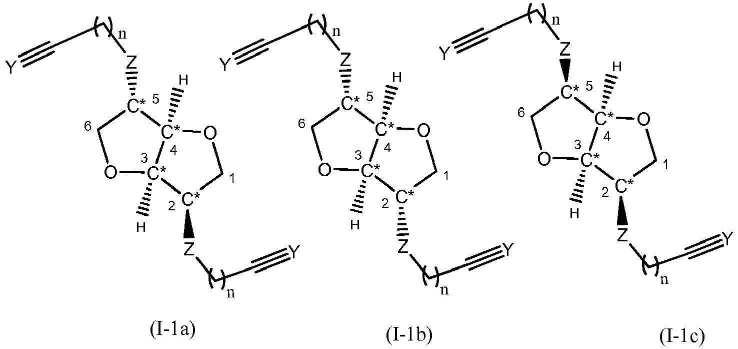 dans lesquelles :
- Z est O, S ou NH, de préférence Z est O,
- n compris entre 1 et 100, de préférence n est compris entre 1 et 10 et encore plus préférentiellement n = 1, et
- Y est CH ou N, de préférence Y est CH.

### Monomères "AB"

Ces monomères selon l'invention sont des monomères de formule I ou I' dans laquelle R¹ et R² sont différents avec :
R¹ représentant un radical 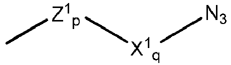
et R² représentant un radical-Z²R³,
   avec :
   - p=0 ou 1,
   - q = 0 ou 1,
   - Z¹, Z² représentant un radical divalent correspondant à : 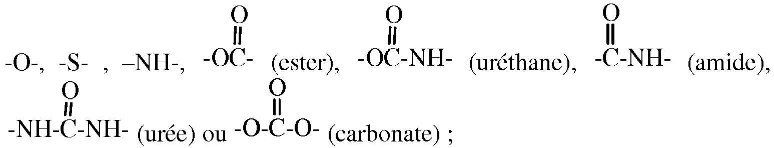
   - R³ étant un radical divalent de formule : -X²-C≡Y ;
   - -X¹-, -X²- étant indépendamment des liaisons covalentes ou des rotules divalentes formées par un groupement (cyclo)aliphatique et/ou aromatique, halogénés ou non et contenant ou non des hétéroatomes, de préférence un groupement -(CH₂)ₙ- avec n compris entre 1 et 100 ;
   - Y représentant CH ou N.

Il s'agit notamment de monomères répondant à la formule développée 1-2 ci-dessous : 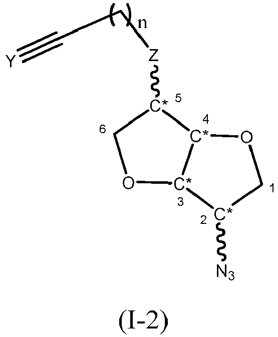 dans laquelle :
- Z est O, S ou NH,
- n compris entre 1 et 100, et
- Y est CH ou N.

De préférence, les monomères de type "AB" de l'invention répondent aux formules développées I-2d à I-2g ci-dessous : 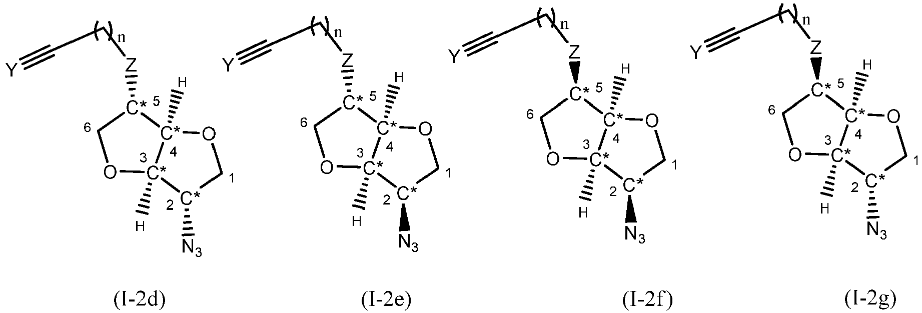 dans lesquelles :
- Z est O, S ou NH, de préférence Z est O,
- n compris entre 1 et 100, de préférence n est compris entre 1 et 10 et encore plus préférentiellement n = 1, et
- Y est CH ou N, de préférence Y est CH.

Selon un mode préféré de l'invention, dans les formules I-1, 1-2, I-1a à I-1c et I-2d à I-2g ci-dessus décrites, Z est un atome d'oxygène.
Dans les formules I, I', I-1, I-2, I-1a à I-1c et I-2d à I-2g ci-dessus décrites, on préfère que n soit compris entre 1 et 10, et plus préférentiellement que n = 1.
Selon un autre mode de réalisation les formules I, I', I-1, 1-2, I-1a à I-1c et I-2d à I-2g ci-dessus décrites, Y est CH.
De préférence les monomères de formules I-1, 1-2, I-1a à I-1c et I-2d à I-2g selon l'invention répondent à l'une des formules I-1, 1-2, I-1a à I-1c ou I-2d à I-2g ci-dessus développées dans laquelle Z est O, n = 1 et Y est CH.

### Monomères "BB"

Les monomères "BB" répondent à la formule I ou I' dans laquelle R¹ et R² sont identiques et représentent un radical 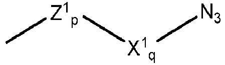 avec :
- p=0 ou 1 ;
- q = 0 ou 1 ;
- Z¹ représente un radical divalent correspondant à : 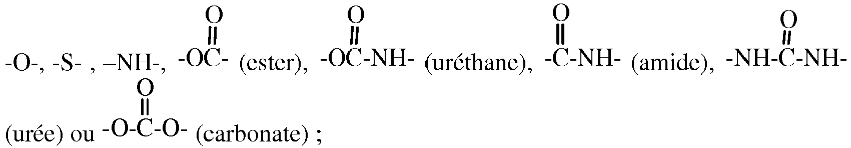
- -X¹- est une liaison covalente ou une rotule divalente formée par un groupement (cyclo)aliphatique et/ou aromatique, halogéné ou non et contenant ou non des hétéroatomes, de préférence un groupement -(CH₂)ₙ- avec n compris entre 1 et 100.

Il s'agit notamment des composés de formule 1-3 :

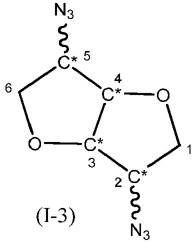

Dans toutes les formules I et I' des monomères "AA", "BB" et "AB" de l'invention, on préfère quand :
- X¹ est une liaison covalente,
- X² est un -(CH₂)ₙ avec 1 ≤ n ≤ 10, de préférence n = 1,
- Z¹ et Z² sont des radicaux -O-, et
- Y est CH.

### Le procédé d'obtention des monomères

La réaction mise en jeu dans le procédé de préparation des monomères tels que définis précédemment dans la description, est notamment une substitution nucléophile. De préférence, au moins deux substitutions nucléophiles sont nécessaires pour obtenir les monomères de l'invention. Ces réactions consistent soit en une dialkylation des positions 2 et 5, soit en une alkylation de la position 2 suivie d'une azidation de la position 5, soit en une azidation de la position 2 suivie d'une alkylation de la position 5.

De manière préférée, les monomères de type "AB" sont réalisés par azidation-alkylation.

Les monomères de type "AA" sont quant à eux réalisés par dialkylation et les monomères de type "BB" sont préparés par diazidation des 2,5-diols 1,4 :3,6-dianhydrohexitols de départ ou de leurs dérivés.

D'une façon générale, le procédé de préparation des monomères de formule I', tels que définis précédemment dans la description, fait intervenir :
- un dérivé 1,4:3,6-dianhydrohexitol de formule II : 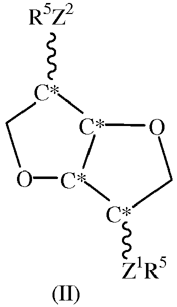 dans laquelle les radicaux R⁵ sont identiques ou différents et représentent un hydrogène ou un alkyle en C1-C10; Z¹, Z² représentant un radical divalent correspondant à : 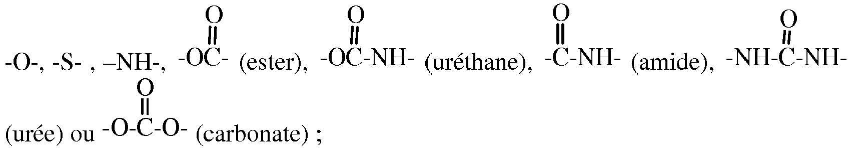 et -Z¹R⁵ et -Z²R⁵ pouvant être par exemple -OH; -NH₂; -SH; -OCH₃ ;
- avec au moins un précurseur des radicaux R1 et/ou R2.

L'homme de l'art est à même de sélectionner les précurseurs des radicaux R¹ et/ou R² et de les mettre en oeuvre dans des réactions chimiques classiques, qui conduiront aux différents types de monomères de formule I'.

Selon un mode particulier de réalisation de l'invention, le procédé de préparation des monomères de formule I' est réalisé à partir de 1,4 :3,6-dianhydrohexitol, de préférence à partir d'isosorbide, d'isomannide ou d'isoidide.

Dans ce cas, -Z¹R⁵ et -Z²R⁵ correspondent chacun à -OH, et les précurseurs des radicaux R¹ et/ou R² peuvent donc être :
- MN₃ ; M correspondant à H ou à un métal, de préférence Na,
   ou
- -Z²R³, OCNR³ ou R⁴Z²R³; avec R⁴ correspondant à H ou un halogène.

Les 1,4 :3,6-dianhydrohexitols ci-dessus sont préparés par double déshydratation de sucres en C₆ (hexitols), de préférence le sorbitol, le mannitol et l'iditol, par exemple à l'aide d'acides forts tels l'acide sulfurique, l'acide chlorhydrique ou l'acide phosphorique, ou à l'aide de catalyseurs acides zéolithes, conformément au schéma réactionnel ci-dessous :

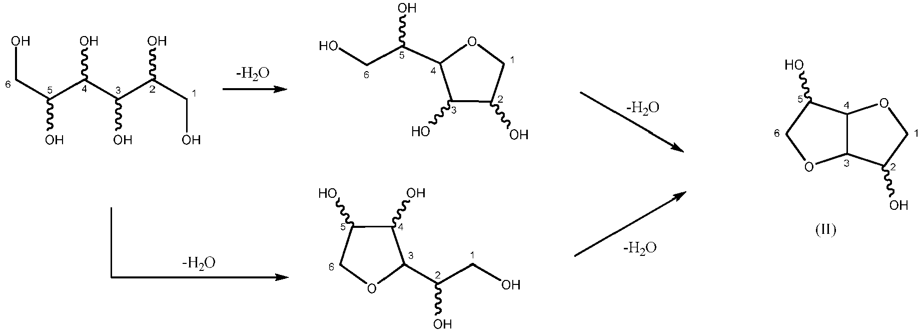

De manière plus détaillée, l'alkylation ou la dialkylation peut être réalisée par exemple à l'aide :
- soit d'une base puis d'un agent alkylant de formule : R⁴Z²R³; avec R⁴ correspondant à H ou un halogène, Z² et R³ étant tels que définis supra.
   Ce type d'alkylation permet de conserver la stéréochimie du carbone chiral ;
- soit d'anhydride triflique (Tf₂O) ou de chlorure de tosyle (Ts-Cl) puis d'un agent alkylant de formule : ⁻Z²R³, avec Z² et R³ étant tels que définis supra.
   Ce type d'alkylation crée une inversion de configuration du site chiral la subissant.

La réaction d'alkylation a avantageusement lieu en solution dans un solvant organique choisi parmi : CH₂Cl₂, THF, DMF, et DMSO. De préférence, le solvant utilisé pour la réaction d'alkylation est le DMF.
La base est préférentiellement choisie dans le groupe des bases fortes de pH dans l'eau supérieur à 8, de préférence supérieur à 10 et encore plus préférentiellement supérieur à 12. De préférence, la base est choisie dans le groupe des bases suivantes : NaOH, NaH et LiH.

Les agents alkylants sont choisis de préférence dans le groupe des composés suivants : le bromure de propargyle, l'alcool propargylique et le chlorure de propargyle.

De façon préférée, l'alkylation est réalisée à l'aide de NaH dans le DMF puis par ajout de bromure de propargyle.

L'azidation ou la diazidation peut être avantageusement réalisée en présence :
- soit de diisopropyl azodicarboxylate, de triphényl phosphine et de HN₃ en excès, conformément à la réaction de Mitsunobu,
- soit d'anhydride triflique (Tf₂O) ou de chlorure de tosyle (Ts-Cl) puis de NaN₃.
Les azidations ainsi réalisées induisent une inversion de la stéréochimie du carbone supportant la substitution.

La réaction d'azidation a avantageusement lieu en solution dans un solvant organique choisi parmi : CH₂Cl₂, THF, DMF, et DMSO. De préférence, le solvant utilisé pour la réaction d'azidation est le DMF.

De façon préférée, l'azidation est réalisée en présence du chlorure de tosyle puis de NaN₃ dans le DMF.

Les substitutions nucléophiles ci-dessus décrites peuvent bien évidemment être réalisées par tout autre moyen connu de l'homme du métier qui consisterait à réaliser une dialkylation, une diazidation, une alkylation-azidation ou encore une azidation-alkylation.

Les monomères I-1, que l'on appelle monomères "AA", sont par exemple synthétisés par dialkylation des 2,5-diols 1,4 :3,6-dianhydrohexitols de départ, selon le schéma réactionnel suivant : 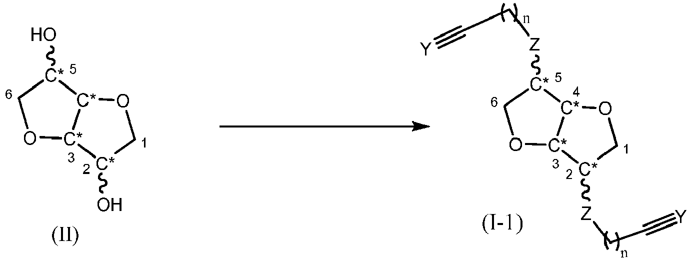
- avec Z est O, NH ou S,
- Y est CH ou N, et
- n est compris entre 1 et 100.

Les monomères de formule 1-2, appelés monomères "AB", peuvent être obtenus soit par une azidation suivie d'une alkylation, soit par une alkylation suivie d'une azidation, à partir des 2,5-diols 1,4 :3,6-dianhydrohexitols de départ.

Selon un premier mode de réalisation du procédé de préparation des monomères de formule 1-2 de type "AB" conformes à l'invention, on réalise une alkylation-azidation selon le schéma réactionnel suivant : 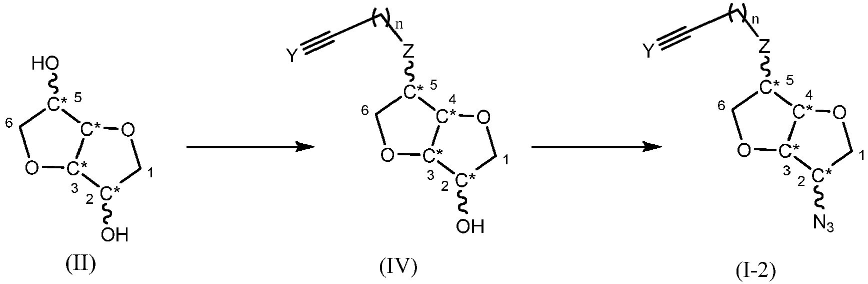
- avec Z est O, NH ou S,
- Y est CH ou N, et
- n est compris entre 1 et 100.

Selon un second mode de réalisation du procédé de préparation des monomères de formule 1-2 de type "AB" conformes à l'invention, on réalise une azidation-alkylation selon le schéma réactionnel suivant : 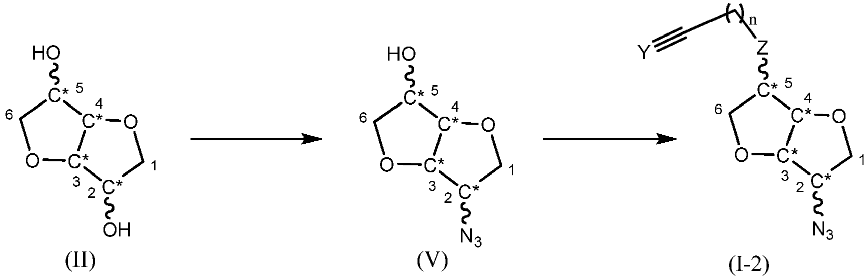
- avec Z est O, NH ou S,
- Y est CH ou N, et
- n est compris entre 1 et 100.

Les monomères de type "BB", répondant à la formule 1-3 sont préparés par diazidation des 2,5-diols 1,4 :3,6-dianhydrohexitols de départ, selon le schéma réactionnel ci-dessous :

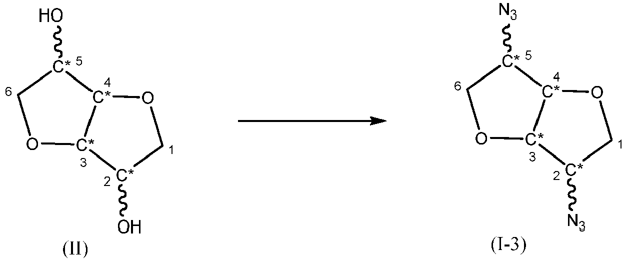

### Les Composés intermédiaires (dimères) obtenus à partir des monomères de formule (I) dans laquelle R¹ = OH

Les composés dimères intermédiaires ont les formules III et III' suivantes : 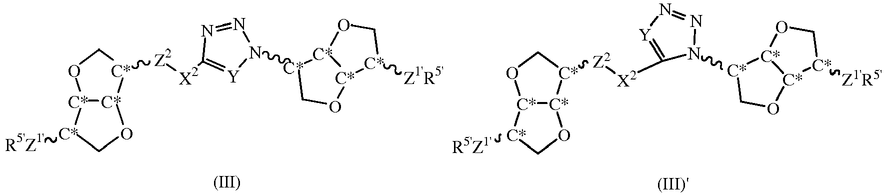 dans lesquelles :
- Y est CH ou N, de préférence CH ;
- Z² représente un -O-, -S- ou -NH-, de préférence -O- ;
- -X²- est -(CH₂)ₙ- avec n compris entre 1 et 100, de préférence 1 ≤ n ≤ 10, et plus préférentiellement n = 1 ;
- Z^{1'} représente un -O-, -S- ou -NH-, de préférence -O- ;
- R^{5'} représente un hydrogène.

Ces composés intermédiaires sont utiles, nouveaux et performants pour l'obtention de polymères à motifs triazole ou tétrazole et riches en motifs 1,4 :3,6-dianhydrohexitol tels que ceux décrits ci-dessous. Ces composés dimères sont réactifs et permettent l'obtention de polymères dans des réactions de polymérisation ultérieures variées, comme par exemple des réactions avec des acides, des esters ou des isocyanates.

### Le procédé d'obtention des composés dimères intermédiaires

Le procédé de préparation des composés dimères intermédiaires de formule III et III' mettent en jeu des monomères monofonctionnels de formules IV et V ci-dessus décrites, par exemple selon le schéma réactionnel ci-dessous :

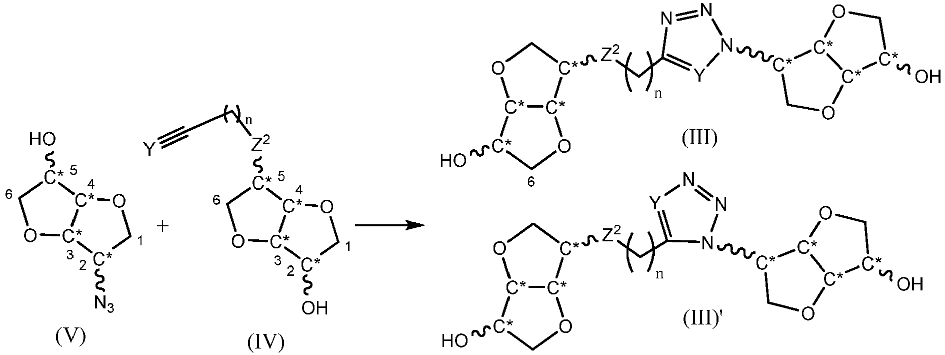

Cette réaction est une réaction de cycloaddition 1,3 dipolaire qui est réalisée de préférence en solution dans un solvant, en présence, de préférence, d'un catalyseur et d'une base.
Le procédé de préparation des composés intermédiaires de formule III a lieu avantageusement par catalyse au cuivre I. Le catalyseur est avantageusement choisi parmi les composés suivants : CuI, CuI.P(OEt)₃, CuI.PPh₃, CuBr.PPh₃. On préfère utiliser CuI.P(OEt)₃. Le procédé de préparation des composés intermédiaires de formule III' a lieu avantageusement par catalyse au ruthénium II. Le catalyseur est avantageusement choisi parmi les composés suivants : Ru(OAc)₂(PPh₃)₂, CpRuCl(PPh₃)₂, Cp*RuCI(PPh₃)₂, Cp*RuCI(COD), avec Cp étant l'abréviation de cyclopentadienyle et COD de cyclooctadiène. On préfère utiliser Cp*RuCl(PPh₃)₂.
Le catalyseur est présent à raison de 0,001 à 0,1 équivalent molaire, de préférence à raison de 0,01 équivalent molaire, pour un équivalent molaire de monomère de formule IV et un équivalent molaire de monomère de formule V.
Selon un mode de réalisation préféré du procédé les monomères de formule IV et de formule V sont introduits en quantité stoechiométrique.

La base est préférentiellement choisie dans le groupe des bases de pKa dans l'eau supérieur à 8, de préférence supérieur à 10.
De préférence, la base est choisie dans le groupe des bases suivantes : DIPEA, NEt₃, 2,6-lutidine.

La base est introduite à raison de 0,5 à 5 équivalents molaire, de préférence de 1 à 3 équivalents molaire, pour un équivalent molaire de monomère de formule IV.
Par ailleurs, la réaction de cycloaddition est préférablement mise en oeuvre dans un milieu réactionnel dont la température est comprise entre 20 et 100 °C, de préférence entre 50 et 80 °C, pendant 0,1 à 48 heures, de préférence pendant 0,5 heure à 36 heures, et plus préférentiellement encore pendant 1 à 15 heures.
Le chauffage du milieu réactionnel est réalisé par tout moyen approprié. L'irradiation micro-ondes peut constituer, e.g. une modalité de chauffage intéressante.
Avantageusement, le milieu réactionnel de la réaction de cycloaddition est un milieu aqueux, hydroorganique ou organique comprenant de préférence au moins un solvant choisi parmi :
- les solvants polaires aprotiques, de préférence le DiMéthylFormamide (DMF), le DMI (DiMéthylIsosorbide), le DiMéthylAcétamide (DMAc), le tétrahydrofurane (THF), l'acétone, la méthyléthylcétone, la butanone ; le DiMéthyl Sulfoxide (DMSO), la N-Méthyl Pyrrolidone (NMP), la DiMéthylEthyl Urée (DMEU),
- les solvants polaires protiques, de préférence le méthanol, l'isopropanol (IPA ou i-PrOH), le t-butanol (t-BuOH),
- les solvants apolaires, de préférence le toluène, l'hexane, le xylène,
- l'eau,
- et leurs mélanges.
Conformément à un mode préféré de réalisation du procédé les monomères sont solubilisés à un taux compris entre 5 et 50 % massique, de préférence entre 10 et 40 % massique dans un solvant choisi parmi les solvants ci-dessus.

### Les Polymères et oligomères

Les dénominations "AB" et "AA/BB" sont des noms arbitraires qui permettent de distinguer les polymères issus d'une part de l'homopolymérisation de monomères de type "AB" et d'autre part de la polymérisation de monomères de type "AA" avec des monomères de type "BB".

Les monomères mis en jeu sont notamment les monomères de formule 1-2 de type "AB", de formule 1-1 de type "AA" et les monomères de formule 1-3 de type "BB" qui sont décrits plus haut dans la description.

Les premiers polymères selon l'invention sont des polymères de type "AB" et répondent à la formule VI suivante : 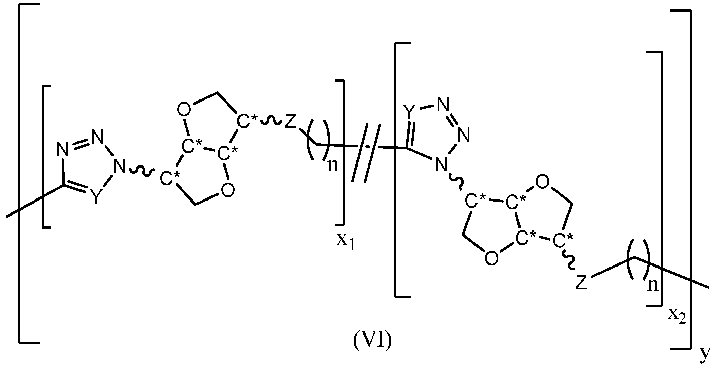 dans laquelle
- y est le degré de polymérisation moyen,
- x₁ et x₂ varient de 0 à 100% en fonction de la présence (ou non) et de la nature du catalyseur,
- n est compris entre 1 et 100,
- Y est CH ou N,
- Z est O, S ou NH.

Le copolymère répondant à la formule VI ci-dessus est de préférence un copolymère statistique.

Le procédé de préparation des polymères de type "AB" tel que défini ci-dessus, est une homopolymérisation par réaction de cycloaddition 1,3 dipolaire mettant en oeuvre un monomère de formule 1-2 de type "AB" tel que défini infra dans la description.

Les seconds polymères, selon l'invention, sont du type "AA/BB" et répondent à la formule VII suivante : 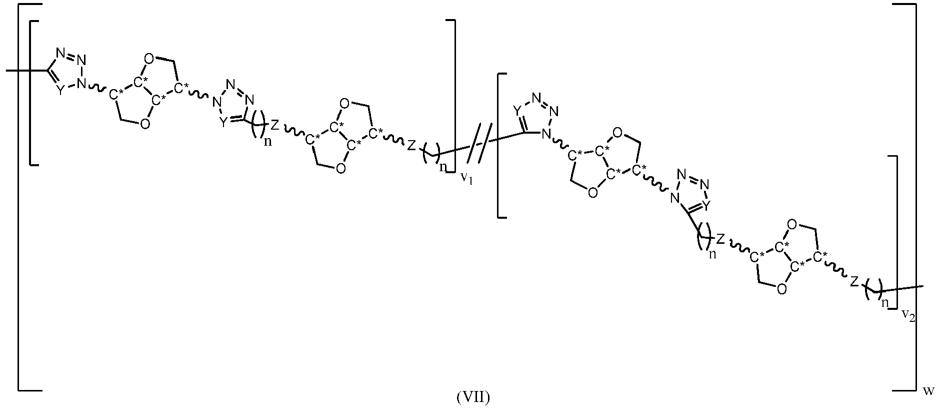 dans laquelle
- w est le degré de polymérisation moyen,
- v₁ et v₂ varient de 0 à 100% en fonction de la présence (ou non) et de la nature du catalyseur,
- n est compris entre 1 et 100,
- Y est CH ou N,
- Z est O, S ou NH.

Le copolymère répondant à la formule VII ci-dessus est de préférence un copolymère statistique.

Selon l'invention, le procédé de préparation du polymère de type "AA/BB" tel que défini plus haut, est une polymérisation par cycloaddition 1,3 dipolaire mettant en oeuvre une quantité stoechiométrique d'un monomère de formule 1-1 de type "AA" tel que défini plus bas dans la description, avec une quantité stoechiométrique d'un monomère de type "BB", répondant à la formule 1-3 ci-dessous : 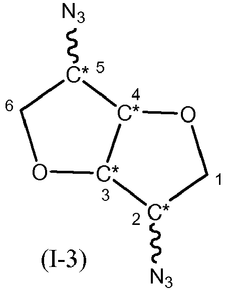 dans laquelle les carbones C3* et C4* sont de préférence de configuration exo (S).

La stratégie de polymérisation "AB", qui utilise un seul type de monomère de type "AB", porteur à la fois des fonctions azoture et alcyne ou nitrile, présente l'avantage de pouvoir s'affranchir des problèmes de contrôle de stoechiométrie.
La stratégie de polymérisation "AA/BB" utilise quant à elle deux types de monomères, l'un de type "AA", et l'autre de type "BB".

Les polymères et oligomères préparés selon ces diverses stratégies possèdent des propriétés spécifiques comme des températures de transition vitreuses élevées, des stabilités thermiques améliorées ou bien encore une aptitude à la filmification et à l'adhésion sur métal tels que le cuivre ou le zinc.

Ces polymères peuvent être mise en forme notamment par extrusion, extrusion-soufflage, ou moulage par injection ou par compression ou par différentes méthodes de coating.

Dans les formules des polymères VI et VII de la présente invention, les terminaisons ne sont pas représentées. L'homme de l'art est à même de les retrouver aisément au vu des monomères utilisés dans leurs procédés de préparation respectifs. Les polymères de l'invention peuvent également être bloqués en bout de chaîne par des termineurs de chaines monofonctionnels de type A ou B et possédant des groupements type : acide, alcool, amine, isocyanate, thiol, époxyde, ces fonctions ayant démontré leur inertie vis à vis de la réaction de cycloaddition.

### Le polymère de type "AB"

Ce polymère est appelé polymère de type "AB" car il est préparé à partir de l'homopolymérisation de monomères de type "AB", par exemple de formule 1-2, c'est-à-dire de monomères porteurs à la fois d'une fonction alcyne ou nitrile et d'une fonction azoture.

Dans la formule VI du polymère "AB", on préfère que :
- Z soit un O,
- Y soit un CH, et
- 1≤ n ≤ 10, de préférence n = 1.

Selon une caractéristique de l'invention, le polymère de type "AB" présente un degré de polymérisation y compris entre 1 et 500, de préférence compris entre 1 et 300 et encore plus préférentiellement compris entre 1 et 200.

Selon une autre caractéristique de l'invention, le polymère de type "AB" présente une température de transition vitreuse Tg_{AB} supérieure à 100 °C, de préférence supérieure à 120 °C et encore plus préférentiellement supérieure à 140 °C.
Une telle température de transition vitreuse du polymère "AB" à motif dianhydrohexitol/ triazole ou tétrazole est nettement supérieures à celle d'un système comparatif benzyle/triazole ou tétrazole, qui présente une Tg inférieure ou égale à 125 °C.

Les polymères de type "AB" selon l'invention sont également caractérisés en ce qu'ils sont riches en motif 1,4 :3,6-dianhydrohexitol, c'est-à-dire qu'ils sont constitués, notamment en fonction de la taille des groupements, à 20 % massique, de préférence à 40 % massique, et encore plus préférentiellement de 60 % massique, de motif 1,4 :3,6-dianhydrohexitol d'origine naturelle.

### Le polymère de type "AA/BB"

Selon une caractéristique de l'invention, le polymère de type "AA/BB" présente un degré de polymérisation w compris entre 1 et 500, de préférence compris entre 1 et 300 et encore plus préférentiellement compris entre 1 et 200.

Selon une autre caractéristique de l'invention, le polymère de type "AA/BB" présente une température de transition vitreuse Tg_{AA/BB} supérieure à 100 °C, de préférence supérieure à 120 °C et encore plus préférentiellement supérieure à 145 °C.
Une telle température de transition vitreuse du polymère "AA/BB" à motif dianhydrohexitol/ triazole ou tétrazole est nettement supérieure à celle d'un système comparatifs isosorbide/ triazole/ benzyl, qui présente une Tg inférieure à 95 °C.

Les polymères de type "AA/BB" selon l'invention sont également caractérisés en ce qu'ils sont riches en motif 1,4:3,6-dianhydrohexitol, c'est-à-dire qu'ils sont constitués, notamment en fonction de la taille des groupements, à 20 % massique, de préférence à 40 % massique, et encore plus préférentiellement de 60 % massique, de motif 1,4 :3,6-dianhydrohexitol d'origine naturelle.

### Les Procédés de préparation des polymères et oligomères de type "AB" et de type "AA/BB"

Selon l'invention, le procédé de préparation des polymères "AB" tels que définis plus haut dans la description ou des polymères de type "AA/BB" tels que définis précédemment dans la description, comprend une cycloaddition 1,3 dipolaire.

Selon le procédé de préparation ci-dessus, la réaction de cycloaddition 1,3 dipolaire est une réaction de polymérisation en masse contrôlée par la température et la pression, en présence ou en absence de catalyseur.

### Le procédé de préparation du polymère de type "AB"

### En solvant :

Selon un premier mode de réalisation du procédé de préparation des polymères de type "AB" conformes à l'invention, la réaction de cycloaddition 1,3-dipolaire a lieu en solution dans un solvant, de préférence en présence d'un catalyseur et d'une base, par exemple selon le schéma suivant : 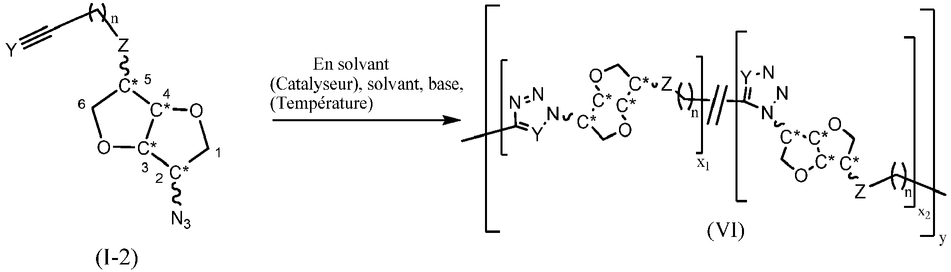 dans laquelle
- y est le degré de polymérisation moyen,
- x₁ et x₂ varient de 0 à 100% en fonction de la présence (ou non) et de la nature du catalyseur,
- n est compris entre 1 et 100,
- Y est CH ou N,
- Z est O, S ou NH.

Dans le schéma ci-dessus, on procède de préférence en présence d'un catalyseur et d'une base, mais il est également possible de réaliser le procédé de l'invention sans catalyseur et sans base en augmentant la température.

Le procédé de préparation en solvant et en présence de catalyseur est régiosélectif, ce qui conduit à un polymère de formule VI dans laquelle x₁ égale 100 % ou x₂ égale 100 %.

Le procédé de préparation des polymères de type "AB" a lieu avantageusement par catalyse au cuivre I ou par catalyse au ruthénium II. Le catalyseur est avantageusement choisi parmi les composés suivants : CuI, CuI.P(OEt)₃, CuI.PPh₃, CuBr.PPh₃, Ru(OAc)₂(PPh₃)₂, CpRuCl(PPh₃)₂, Cp*RuCl(PPh₃)₂, Cp*RuCl(COD). On préfère utiliser CuI.P(OEt)₃ ou Cp*RuCI(PPh₃)₂.
Le catalyseur est présent à raison de 0,001 à 0,1 équivalent molaire, de préférence à raison de 0,01 équivalent molaire, pour un équivalent molaire de monomère de type "AB" de formule 1-2.

La base est préférentiellement choisie dans le groupe des bases de pKa dans l'eau supérieur à 8, de préférence supérieur à 10.

De préférence, la base est choisie dans le groupe des bases suivantes : DIPEA, NEt₃, 2,6-lutidine.
Selon l'invention, la base est introduite à raison de 0,5 à 2,5 équivalents molaire, de préférence de 1 à 2 équivalents molaire, pour un équivalent molaire de monomère de type "AB" de formule 1-2.

Par ailleurs, la réaction de cycloaddition est préférablement mise en oeuvre dans un milieu réactionnel dont la température est comprise entre 20 et 100 °C, de préférence entre 50 et 80 °C, pendant 0,1 à 48 heures, de préférence pendant 0,5 heure à 36 heures, et plus préférentiellement encore pendant 1 à 15 heures.
Le chauffage du milieu réactionnel est réalisé par tout moyen approprié. L'irradiation micro-ondes peut constituer, e.g. une modalité de chauffage intéressante.

Avantageusement, le milieu réactionnel de la réaction de cycloaddition est un milieu aqueux, hydroorganique ou organique comprenant de préférence au moins un solvant choisi parmi :
- les solvants polaires aprotiques, de préférence le DiMéthylFormamide (DMF), le DMI (DiMéthylIsosorbide), le DiMéthylAcétamide (DMAc), le tétrahydrofurane (THF),l'acétone, la méthyléthylcétone, la butanone ; le DiMéthyl Sulfoxide (DMSO), la N-Méthyl Pyrrolidone (NMP), la DiMéthylEthyl Urée (DMEU),
- les solvants polaires protiques, de préférence le méthanol, l'isopropanol (IPA ou i-PrOH), le t-butanol (t -BuOH),
- les solvants apolaires, de préférence le toluène, l'hexane, le xylène,
- l'eau,
- et leurs mélanges.

Conformément à un mode préféré de réalisation du procédé de l'invention, le monomère est solubilisé à un taux compris entre 5 et 50 % massique, de préférence entre 10 et 40 % massique dans un solvant choisi parmi les solvants ci-dessus.

Cette réaction de cycloaddition 1,3 dipolaire est généralement suivie d'une précipitation puis d'un lavage et d'un séchage sous pression réduite.
La précipitation est effectuée de préférence dans l'éther diéthylique si le solvant utilisé pour la réaction est du DMF, et dans l'éthanol pour les autres solvants.
Le lavage est réalisé avantageusement à l'eau puis à l'acétone, et le séchage est un séchage sous pression réduite.

### En masse :

Selon un deuxième mode de réalisation du procédé de préparation des polymères de type "AB" conformes à l'invention, la réaction de cycloaddition 1,3-dipolaire est une réaction de polymérisation en masse.
On entend *e.g.* par réaction en masse au sens du présent exposé, une réaction de cycloaddition 1,3-dipolaire entre des monomères de type "AB" de formule 1-2 dans des conditions de température et de pression contrôlées, éventuellement en présence d'un catalyseur et d'une base.
Selon un mode de réalisation particulier du procédé de polymérisation en masse ci-dessus, la réaction de polymérisation a lieu en l'absence de catalyseur. Ce mode particulier de préparation des polymères AB de l'invention présente l'avantage de conférer des températures de transition vitreuse du même ordre qu'en présence de catalyseur. En outre un tel mode de préparation est simple à mettre en oeuvre, économique et respectueux de l'environnement puisqu'il ne nécessite la mise en oeuvre d'aucun autre réactif que les monomères AB. 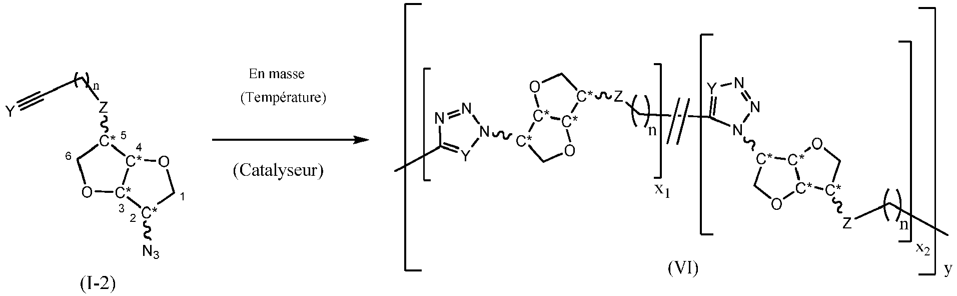 dans laquelle
- y est le degré de polymérisation moyen,
- x₁ et x₂ varient de 0 à 100% en fonction de la présence (ou non) et de la nature du catalyseur,
- n est compris entre 1 et 100,
- Y est CH ou N,
- Z est O, S ou NH.

S'il est utilisé, le catalyseur est avantageusement choisi parmi les composés suivants : CuI, CuI.P(OEt)₃, CuI.PPh₃, CuBr.PPh₃, Ru(OAc)₂(PPh₃)₂, CpRuCI(PPh₃)₂, Cp*RuCI(PPh₃)₂, Cp*RuCl(COD), avec Cp étant le cyclopentadienyle et COD étant le cyclooctadiène. On préfère utiliser CuI.P(OEt)₃ ou Cp*RuCl(PPh₃)₂.
Le catalyseur est présent à raison de 0,001 à 0,1 équivalent molaire, de préférence à raison de 0,01 équivalent molaire, pour un équivalent molaire de monomère de type "AB" de formule 1-2.
Si elle est utilisée, la base est préférentiellement choisie dans le groupe des bases de pKa dans l'eau supérieur à 8, de préférence supérieur à 10.
De préférence, la base est choisie dans le groupe des bases suivantes : DIPEA, NEt₃, 2,6-lutidine.
Selon l'invention, la base est introduite à raison de 0,5 à 2,5 équivalents molaire, de préférence de 1 à 2 équivalents molaire, pour un équivalent molaire de monomère de type "AB" de formule 1-2.

Ce procédé de préparation aboutit à un copolymère statistique de formule VI ci-dessus. Il est particulièrement avantageux de constater que cette réaction peut avoir lieu sans activation thermique ni catalytique.

Les conditions de température et de pression dans lesquelles le monomère de type "AB" est placé permettent de contrôler le degré de polymérisation y du copolymère obtenu et donc sa température de transition vitreuse. Ainsi, une grande variété de copolymères peut être obtenue par ce procédé.

Par exemple, un monomère placé à température ambiante (25 °C) atteindra, au bout d'un temps t variable selon le type de monomère "AB", un degré de polymérisation y moyen d'environ 5. Sa température de transition vitreuse sera d'environ 25 °C. Un tel composé oligomère réactif est susceptible d'être transformé en polymère de degré de polymérisation supérieur par simple augmentation de la température, par exemple 30 secondes à 200 °C. Dans un tel cas d'exemple, la température de transition vitreuse du polymère obtenu in fine est de l'ordre de 140 °C à 165 °C.

### Le procédé de préparation du polymère de type AA/BB

### En solvant :

Selon un deuxième mode de réalisation du procédé de préparation des polymères de type "AA/BB" conformes à l'invention, la réaction de cycloaddition 1,3-dipolaire a lieu en solution dans un solvant, de préférence en présence d'un catalyseur et d'une base, par exemple selon le schéma suivant : 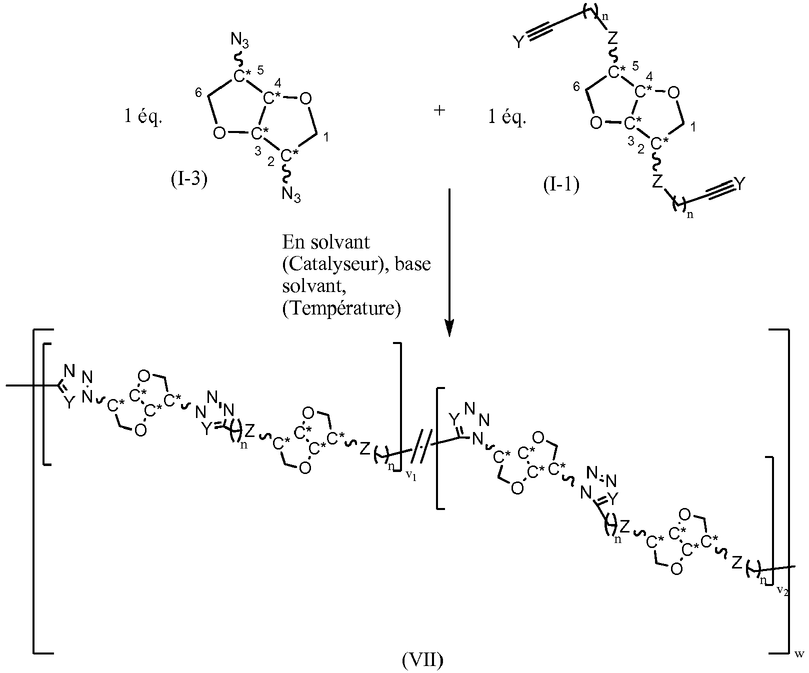 dans laquelle
- w est le degré de polymérisation moyen,
- v₁ et v₂ varient de 0 à 100% en fonction de la présence (ou non) et de la nature du catalyseur,
- n est compris entre 1 et 100,
- Y est CH ou N,
- Z est O, S ou NH.

Dans le schéma réactionnel ci-dessus, on procède de préférence en présence de catalyseur et d'une base, mais il est également possible de réaliser le procédé de l'invention sans catalyseur et sans base, en augmentant la température.

Ce procédé de préparation en solvant et en présence de catalyseur est régiosélectif, ce qui conduit à un polymère de formule VII dans laquelle v₁ égale 100 % ou v₂ égale à 100 %. Le procédé de préparation des polymères de type "AA/BB" a lieu avantageusement par catalyse au cuivre I ou par catalyse au ruthénium II. Le catalyseur est avantageusement choisi parmi les composés suivants : CuI, CuI.P(OEt)₃, CuI.PPh₃, CuBr.PPh₃, Ru(OAc)₂(PPh₃)₂, CpRuCl(PPh₃)₂, Cp*RuCl(PPh₃)₂, Cp*RuCl(COD), avec Cp étant le cyclopentadienyle et COD étant le cyclooctadiène. On préfère utiliser CuI.P(OEt)₃ ou Cp*RuCl(PPh₃)₂.
Le catalyseur est présent à raison de 0,001 à 0,1 équivalent molaire, de préférence à raison de 0,01 équivalent molaire, pour un équivalent molaire de monomère de type "AA" de formule I-1 et un équivalent molaire de monomère de type "BB" de formule I-3.
Selon un mode de réalisation préféré du procédé de l'invention, les monomères de type "AA" de formule 1-1 et de type "BB" de formule 1-3 sont introduits en quantité stoechiométrique.

La base est préférentiellement choisie dans le groupe des bases de pKa dans l'eau supérieur à 8, de préférence supérieur à 10.

De préférence, la base est choisie dans le groupe des bases suivantes : DIPEA, NEt₃, 2,6-lutidine.
Selon l'invention, la base est introduite à raison de 0,5 à 5 équivalents molaire, de préférence de 1 à 3 équivalents molaire, pour un équivalent de monomère de type "AA" de formule I-1.

Par ailleurs, la réaction de cycloaddition est préférablement mise en oeuvre dans un milieu réactionnel dont la température est comprise entre 20 et 100 °C, de préférence entre 50 et 80 °C, pendant 0,1 à 48 heures, de préférence pendant 0,5 heure à 36 heures, et plus préférentiellement encore pendant 1 à 15 heures.
Le chauffage du milieu réactionnel est réalisé par tout moyen approprié. L'irradiation micro-ondes peut constituer, e.g. une modalité de chauffage intéressante.

Avantageusement, le milieu réactionnel de la réaction de cycloaddition est un milieu aqueux, hydroorganique ou organique comprenant de préférence au moins un solvant choisi parmi :
- les solvants polaires aprotiques, de préférence le DiMéthylFormamide (DMF), le DMI (DiMéthylIsosorbide), le DiMéthylAcétamide (DMAc), le tétrahydrofurane (THF), l'acétone, la méthyléthylcétone, la butanone ; le DiMéthyl Sulfoxide (DMSO), la N-Méthyl Pyrrolidone (NMP), la DiMéthylEthyl Urée (DMEU),
- les solvants polaires protiques, de préférence le méthanol, l'isopropanol (i-PrOH ou IPA), le t-butanol (t-BuOH),
- les solvants apolaires, de préférence le toluène, l'hexane, le xylène,
- l'eau,
- et leurs mélanges.

Conformément à un mode préféré de réalisation du procédé de l'invention, les monomères sont solubilisés à un taux compris entre 5 et 50 % massique, de préférence entre 10 et 40 % massique dans un solvant choisi parmi les solvants ci-dessus.

Cette réaction de cycloaddition 1,3 dipolaire est généralement suivie d'une précipitation puis d'un lavage et d'un séchage sous pression réduite.
La précipitation est effectuée de préférence dans l'éther diéthylique si le solvant utilisé pour la réaction est du DMF, et dans l'éthanol pour les autres solvants.
Le lavage est réalisé avantageusement à l'eau puis à l'acétone, et le séchage est un séchage sous pression réduite.

### En masse :

Selon un deuxième mode de réalisation du procédé de préparation des polymères de type "AA/BB" conformes à l'invention, la réaction de cycloaddition 1,3-dipolaire est une réaction de polymérisation en masse.
On entend par réaction en masse e.g. une réaction de cycloaddition 1,3-dipolaire entre des monomères de type "AA" par exemple de formule 1-1 et des monomères de type "BB" par exemple de formule 1-3 dans des conditions de température et de pression contrôlées, éventuellement en présence d'un catalyseur et d'une base. Il est particulièrement avantageux de constater que cette réaction peut avoir lieu sans activation thermique ni catalytique.

Selon un mode de réalisation particulier du procédé de polymérisation en masse ci-dessus, la réaction de polymérisation a lieu en l'absence de catalyseur. Ce mode particulier de préparation des polymères "AA/BB" de l'invention présente l'avantage de conférer des températures de transition vitreuse du même ordre qu'en présence de catalyseur. En outre un tel mode de préparation est simple à mettre en oeuvre, économique et respectueux de l'environnement puisqu'il ne nécessite la mise en oeuvre d'aucun autre réactif que les monomères "AA" et "BB". 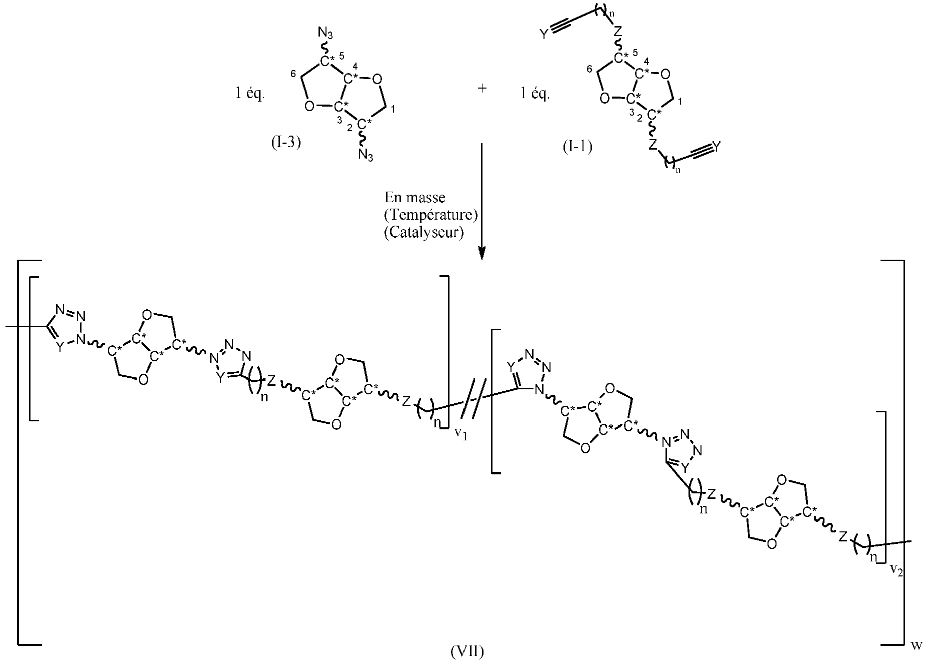 dans laquelle
- w est le degré de polymérisation moyen,
- v₁ et v₂ varient de 0 à 100% en fonction de la présence (ou non) et de la nature du catalyseur,
- n est compris entre 1 et 100,
- Y est CH ou N,
- Z est O, S ou NH.

S'il est utilisé, le catalyseur est avantageusement choisi parmi les composés suivants : CuI, CuI.P(OEt)₃, CuI.PPh₃, CuBr.PPh₃, Ru(OAc)₂(PPh₃)₂, CpRuCl(PPh₃)₂, Cp*RuCl(PPh₃)₂, Cp*RuCl(COD), avec Cp étant le cyclopentadienyle et COD étant le cyclooctadiène. On préfère utiliser CuI.P(OEt)₃ ou Cp*RuCl(PPh₃)₂.
Le catalyseur est présent à raison de 0,001 à 0,1 équivalent molaire, de préférence à raison de 0,01 équivalent molaire, pour un équivalent molaire de monomère de type "AB" de formule 1-2.
Si elle est utilisée, la base est préférentiellement choisie dans le groupe des bases de pKa dans l'eau supérieur à 8, de préférence supérieur à 10.
De préférence, la base est choisie dans le groupe des bases suivantes : DIPEA, NEt₃, 2,6-lutidine.
Selon l'invention, la base est introduite à raison de 0,5 à 2,5 équivalents molaire, de préférence de 1 à 2 équivalents molaire, pour un équivalent molaire de monomère de type "AA" de formule 1-1.

Ce procédé de préparation aboutit à un copolymère statistique de formule VII.

Les conditions de température et de pression dans lesquelles les monomères sont placés permettent de contrôler le degré de polymérisation w du copolymère obtenu et donc sa température de transition vitreuse.

### Applications :

Les monomères, dimères, oligomères et polymères selon l'invention peuvent être utilisés dans de nombreuses applications, à l'instar de leurs homologues connus. Leur provenance de ressources biologiques renouvelables n'est pas le moindre de leurs atouts. L'invention concerne ainsi en particulier :
- un procédé de mise en forme d'un matériau polymère notamment par extrusion, d'extrusion-soufflage, ou de moulage par injection ou par compression, dans lequel le matériau polymère mis en oeuvre est choisi parmi les polymères tels que définis ci-dessus ;
- et un procédé de mise en forme d'un matériau polymère notamment par extrusion, d'extrusion-soufflage, ou de moulage par injection ou par compression, dans lequel le matériau polymère est obtenu, au moins en partie lors de la mise en forme, par polymérisation des monomères et/ou des composés dimères intermédiaires tels que définis précédemment. Par exemple, il peut s'agir d'un procédé de mise en forme par compression dans lequel on introduit, dans une presse, des monomères AB et de la fibre de verre pour obtenir un composite polymère de forme déterminée ;
- un procédé de revêtement d'un support par un polymère tel que défini ci-dessus.

D'autres détails de l'invention apparaîtront plus clairement au vu des exemples donnés ci-dessous à titre indicatif.

### EXEMPLES

### 1. Matériels et techniques de caractérisation utilisés :

### 1. a. La Résonance Magnétique Nucléaire :

Les analyses RMN ont été réalisées sur des spectromètres Bruker^{®} (ALS 300^{®} et DRX 300^{®}) à 75 MHz pour la RMN ¹³C et à 300 MHz pour la RMN ¹H. Des analyses bidimensionnelles (COSY, HMBC, HSQC) ont été réalisées lorsqu'elles étaient nécessaires pour l'attribution des signaux. Pour la RMN ¹H, le pic résiduel du solvant est choisi comme référence pour étalonner le spectre (DMSO-d₆ : 2,50 ppm et CD₃Cl : 7,26 ppm). Les déplacements chimiques sont exprimés en ppm. Les abréviations utilisées dans la description des spectres sont les suivantes : d (doublet), t (triplet) et m (multiplet). Pour la description des spectres RMN ¹³C, la calibration des déplacements chimiques est centrée sur le pic résiduel du solvant (DMSO-d₆ : 39,52 ppm et CD₃Cl : 77,16 ppm).

### 1. b. La Spectrométrie de Masse Haute Résolution :

Les spectres de masse ont été réalisés sur un spectromètre ThermoFinnigan^{®} par ionisation chimique ou électronique au Centre de Spectrométrie de Masse de l'Université Claude Bernard Lyon 1.

### 1. c. L'Analyse par Calorimétrie différentielle « Differential Scanning Calorimetry » :

Les analyses DSC ont été réalisées sur un appareil DSC 2920^{®} (TA Instrument^{®}) en chauffant à une vitesse de 20 °C/min sous atmosphère inerte (hélium ou azote).

### 1. d. L'Analyse ThermoGravimétrique :

Les analyses ATG ont été réalisées sur un appareil TGA 2950^{®} (TA Instrument^{®}) en chauffant à une vitesse de 10 °C/min sous atmosphère inerte (hélium ou azote).

### 1. e. L'Analyse par Chromatographie d'exclusion stérique :

Les analyses SEC ont été réalisées, à 120°C, sur un appareil Waters alliance GPVC 2000 avec un détecteur d'indice de réfraction et trois colonnes PL gel-mixed (Styragel HT2-HT4-HT6). Le solvant d'élution est le DMSO éluant à une vitesse de 1 mL /min.

### 2. Synthèse des diazotures "BB" :

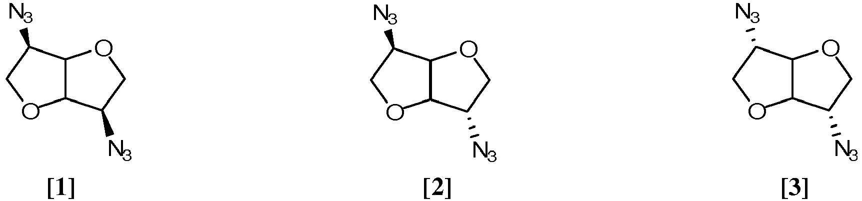

Les diazotures **[2]** et **[3]** sont synthétisés selon la procédure décrite par Thiem (Makromol. Chem. 187, 2775-2785, 1986). Le diazoture **[1]** est synthétisé selon la procédure décrite dans l'exemple 1 ci-dessous.

### Exemple 1 : synthèse du 1,4:3,6-Dianhydro-2,5-diazido-2,5-dideoxy-D-mannitol [1]

A température ambiante et sous argon, de l'isoidide (fourni par ROQUETTE FRERES) (2,0 g, 13,7 mmol) est dissous dans du dichlorométhane anhydre (40 mL) puis de la pyridine (commercialisée par Aldrich^{®} sous le nom pyridine) (11 mL, 137 mmol) est additionnée. La solution est abaissée à 0 °C puis de l'anhydride triflique (commercialisé par Acros organic sous le nom trifluoromehtane sulfonic anhydride 98 %) (5,75 g, 34,3 mmol) est ajouté. Après 20 minutes d'agitation à température ambiante, l'excès de pyridine est neutralisé par l'addition d'une solution d'acide chlorhydrique 1N puis le milieu est lavé par une solution de chlorure d'ammonium saturée (2 x 40 mL). La phase organique est ensuite séchée sur sulfate de magnésium puis concentrée sous pression réduite. L'intermédiaire obtenu (ditriflate) est alors dissous dans du diméthylformamide (50 mL) puis de l'azoture de sodium (commercialisé par Alfa Aesar sous le nom sodium azoture 99 %) (5,4 g, 82, 2 mmol) est additionné. La réaction est poursuivie à température ambiante pendant 4h. Le milieu réactionnel est ensuite dilué par addition d'eau (200 mL) puis extrait avec de l'acétate d'éthyle (2 x 150 mL). La phase organique est séchée sur sulfate de magnésium, concentrée sous pression réduite et le mélange brut est chromatographié sur gel de silice avec comme éluant le mélange heptane / acétate d'éthyle (80 / 20).
Le diazoture **[1]** est obtenu sous la forme d'une huile. Le rendement est de 34 %.

### 3. Synthèse des dialcynes "AA" :

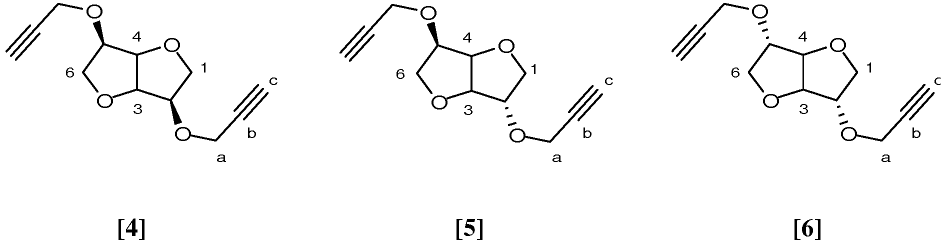

Les dialcynes sont synthétisés selon les procédés décrits dans les exemples 2-4 ci-dessous.

### Exemple 2 : synthèse du 1,4:3,6-Dianhydro-2,5-di-O-propargyl-D-mannitol [4]

A température ambiante et sous argon, de l'isomannide (fourni par ROQUETTE FRERES) (0,5 g, 3,4 mmol) est dissous dans du tétrahydrofurane anhydre (10 mL). La solution est abaissée à 0 °C puis de l'hydrure de sodium à 60 % dans la paraffine (commercialisé par Aldrich^{®} sous le nom sodium hydride 60 % dispersion in mineral oil) (0,68 g, 17 mmol) et une quantité catalytique d'éther de couronne (18-crown-6, commercialisé par Aldrich^{®} sous le nom 18-crown-6 99 %) sont additionnés. Au retour à température ambiante, le bromure de propargyle à 80 % dans du toluène (commercialisé par Aldrich^{®} sous le nom propargyl bromide 80 % wt in toluene) (1,9 mL, 17 mmol) est ajouté. La réaction est poursuivie à température ambiante pendant 4h. L'excès d'hydrure de sodium est neutralisé par addition d'eau (5 mL) puis le tétrahydrofurane est évaporé sous pression réduite. Le résidu obtenu est repris dans 50 mL d'eau et le dialcyne brut ainsi obtenu est extrait avec du dichlorométhane (3 x 30 mL). La phase organique est ensuite séchée sur sulfate de magnésium, concentrée sous pression réduite et le mélange brut est chromatographié sur gel de silice avec comme éluant le mélange éther de pétrole / acétate d'éthyle (80 / 20).
Le dialcyne **[4]** est obtenu sous forme d'huile avec un rendement de 96 %.

### Exemple 3 : synthèse du 1,4:3,6-Dianhydro-2,5-di-O-propargyl-D-sorbitol [5]

A température ambiante et sous argon, de l'isosorbide (fourni par ROQUETTE FRERES) (0,5 g, 3,4 mmol) est dissous dans du tétrahydrofurane anhydre (10 mL). La solution est abaissée à 0 °C puis de l'hydrure de sodium à 60 % dans la paraffine (commercialisé par Aldrich^{®} sous le nom sodium hydride 60 % dispersion in mineral oil) (0,68 g, 17 mmol) et une quantité catalytique d'éther de couronne (18-crown-6, commercialisé par Aldrich^{®} sous le nom 18-crown-6 99 %) sont additionnés. Au retour à température ambiante, le bromure de propargyle à 80 % dans du toluène (commercialisé par Aldrich^{®} sous le nom propargyl bromide 80 % wt in toluene) (1,9 mL, 17 mmol) est ajouté. La réaction est poursuivie à température ambiante pendant 4h. L'excès d'hydrure de sodium est neutralisé par addition d'eau (5 mL) puis le tétrahydrofurane est évaporé sous pression réduite. Le résidu obtenu est repris dans 50 mL d'eau et le dialcyne brut ainsi obtenu est extrait avec du dichlorométhane (3 x 30 mL). La phase organique est ensuite séchée sur sulfate de magnésium, concentrée sous pression réduite et le mélange brut est chromatographié sur gel de silice avec comme éluant le mélange éther de pétrole / acétate d'éthyle (80/20).
Le dialcyne **[5]** est obtenu sous forme d'huile avec un rendement de 96 %.

### Exemple 4 : synthèse du 1,4:3,6-Dianhydro-2,5-di-O-propargyl-D-iditol [6]

A température ambiante et sous argon, de l'isoidide (fourni par ROQUETTE FRERES) (0,5 g, 3,4 mmol) est dissous dans du tétrahydrofurane anhydre (10 mL). La solution est abaissée à 0 °C puis de l'hydrure de sodium à 60 % dans la paraffine (commercialisé par Aldrich^{®} sous le nom sodium hydride 60 % dispersion in mineral oil) (0,68 g, 17 mmol) et une quantité catalytique d'éther de couronne (18-crown-6, commercialisé par Aldrich^{®} sous le nom 18-crown-6 99 %) sont additionnés. Au retour à température ambiante, le bromure de propargyle à 80 % dans du toluène (commercialisé par Aldrich^{®} sous le nom propargyl bromide 80 % wt in toluene) (1,9 mL, 17 mmol) est ajouté. La réaction est poursuivie à température ambiante pendant 4h. L'excès d'hydrure de sodium est neutralisé par addition d'eau (5 mL) puis le tétrahydrofurane est évaporé sous pression réduite. Le résidu obtenu est repris dans 50 mL d'eau et le dialcyne brut ainsi obtenu est extrait avec du dichlorométhane (3 x 30 mL). La phase organique est ensuite séchée sur sulfate de magnésium, concentrée sous pression réduite et le mélange brut est chromatographié sur gel de silice avec comme éluant le mélange éther de pétrole / acétate d'éthyle (80 / 20).
Le dialcyne **[6]** est obtenu sous forme d'huile avec un rendement de 98 %.

### Caractérisation des dialcynes"AA":

**1,4:3,6-Dianhydro-2,5-di-*O*-propargyl-D-mannitol [4]** : RMN ¹H (C₂D₆SO, 300MHz) : δ 4,52-4,53 (m, 2H, H-3, H-4) ; 4,12-4,29 (m, 6H, H-2, H-5, H-a) ; 3,88-3,93 (m, 2H, H-1a, H-6a) ; 3,43-3,48 (m, 4H, H-1b, H-6b, H-c). RMN ¹³C (C₂D₆SO, 75MHz) : δ 80,0 (C-3, C-4) ; 79,7 (C-b) ; 78,4 (C-2, C-5) ; 77,3 (C-c) ; 70,1 (C-1, C-6) ; 56,8 (C-a). MSHR: *m*/*z* calculé pour C₁₂H₁₄O₆ (m/z+H) : 223,0970 ; trouvé : 223,0970.

**1,4:3,6-Dianhydro-2,5-di-*O*-propargyl-D-sorbitol [5]** : RMN ¹H (C₂D₆SO, 300 MHz) : δ 4,57 (t, 1H, *J* =4,6 Hz, H-3) ; 4,47 (d, 1H, *J* = 4,6 Hz, H-4) ; 4,05-4,30 (m, 6H, H-2, H-5, C-a) ; 3,71-3,90 (m, 3H, H-1a, H-6a, H-6b) ; 3,43-3,47 (m, 3H, H-1b, H-c). RMN ¹³C (C₂D₆SO, 75 MHz) : δ 85,1 (C-3) ; 82,5 (C-2) ; 80,0 (C-b) ; 79,7 (C-4) ; 78,4 (C-5) ; 77,3 (C-c) ; 72,3 (C-1) ; 69,4 (C-6) ; 55,9-56,8 (C-a). MSHR: *m*/*z* calculé pour C₁₂H₁₄O₆ (m/z+H) : 223,0970 ; trouvé : 223,0970.

**1,4:3,6-Dianhydro-2,5-di-*O*-propargyl-L-iditol [6]** : RMN¹H (C₂D₆SO, 300 MHz) : δ 4,52 (s1, 2H, H-3, H-4) ; 4,22 (d, 4H, *J* = 2,4 Hz, H-a) ; 4,07-4,08 (m, 2H, H-2, H-5) ; 3,68-3,82 (m, 4H, H-1a, H-1b, H-6a, H-6b) ; 3,46 (t, 2H, *J* = 2,4 Hz, H-c). RMN ¹³C (C₂D₆SO, 75 MHz) : δ 84,4 (C-3, C-4) ; 81,9 (C-2, C-5) ; 80,0 (C-b) ; 77,3 (C-c) ; 71,1 (C-1, C-6) ; 56,0 (C-a). MSHR: *m*/*z* calculé pour C₁₂H₁₄O₆ (m/z+H) : 223,0968 ; trouvé : 223,0968.

### 4. Synthèse des monoalcynes :

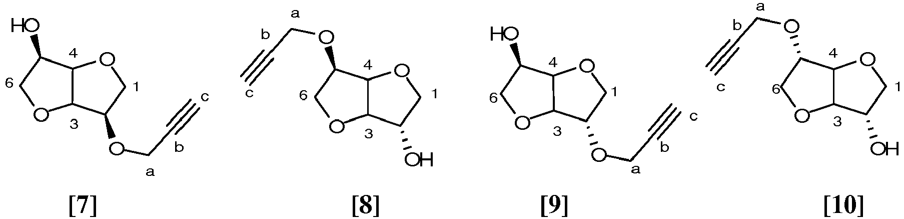

Les monoalcynes sont obtenus soit en présence d'un solvant organique (exemple 5), soit en milieu aqueux (exemple 6).

### Exemple 5 : synthèse du 1,4:3,6-Dianhydro-5-O-propargyl-D-sorbitol [8]

Sous argon, à 90 °C, de l'isosorbide (fourni par ROQUETTE FRERES) (10,0 g, 68,5 mmol) et le chlorure de lithium (2,9 g, 68,5 mmol) sont dissous dans du diméthylsulfoxyde (50 mL) puis de l'hydrure de lithium (commercialisé par Fluka^{®} sous le nom lithium hydride) (0,5 g, 68,5 mmol) est additionné. Après 30 minutes de réaction, le bromure de propargyle à 80 % dans du toluène (commercialisé par Aldrich^{®} sous le nom propargyl bromide 80 % wt in toluene) (7,6 mL, 68,5 mmol) est ajouté. La réaction est poursuivie à 90 °C pendant 2h. Au retour à température ambiante, l'excès d'hydrure de lithium est neutralisé par addition d'eau (15 mL) puis d'acide chlorhydrique 1N (5 mL). Le milieu réactionnel est ensuite dilué avec 300 mL d'eau puis est extrait avec de l'acétate d'éthyle (3 x 250 mL). La phase organique est ensuite séchée sur sulfate de magnésium, concentrée sous pression réduite et le mélange brut est chromatographié sur gel de silice avec comme éluant le mélange heptane / acétate d'éthyle (50 / 50).
Le monoalcyne **[8]** est obtenu sous forme d'huile avec un rendement de 42 %.

### Exemple 6 : synthèse du 1,4:3,6-Dianhydro-5-O-propargyl-L-iditol [10]

Sous agitation, à 75 °C, de l'isoidide (fourni par ROQUETTE FRERES) (10,0 g, 68,5 mmol) et de l'hydroxyde de sodium (2,8 g, 68,5 mmol) sont dissous dans l'eau (65 mL). Le bromure de propargyle à 80 % dans du toluène (commercialisé par Aldrich^{®} sous le nom propargyl bromide 80 % wt in toluene) (7,6 mL, 68,5 mmol) est ensuite ajouté. La réaction est poursuivie à 75 °C pendant 2h30. Au retour à température ambiante, le milieu réactionnel est extrait avec de l'acétate d'éthyle (2 x 80 mL). La phase organique est ensuite séchée sur sulfate de magnésium, concentrée sous pression réduite et le mélange brut est chromatographié sur gel de silice avec comme éluant le mélange heptane / acétate d'éthyle (50 / 50).
Le monoalcyne **[10]** est obtenu sous forme d'huile avec un rendement de 46 %.

Les monoalcynes **[7], [8], [9]** et **[10]** ont également été synthétisés par les procédés ci-dessus décrits. Les résultats obtenus sont récapitulés dans le tableau ci-dessous.

| **Monoalcyne** | **[7]** | **[8]** | **[9]** | **[10]** |
|---|---|---|---|---|
| **Réactif inital** | isomannide | isosorbide | isosorbide | isoidide |
| **Procédé** | A | B | A | A |
| **Rendement (%)*** | 38 | 42 | 8 | 46 |

| | | | | |
|---|---|---|---|---|
| A : procédé décrit dans l'exemple 6 B : procédé décrit dans l'exemple 5 * rendement molaire calculé à partir de l'isosorbide, isomannide ou isoidide. | | | | |

### Caractérisation des monoalcynes :

**1,4:3,6-Dianhydro-2-*O*-propargyl-D-mannitol** [7] : RMN¹H (CDCl₃, 300 MHz) : δ 4,59 (t, 1H, *J* = 4,8 Hz, H-3) ; 4,52 (t, 1H, *J* = 4,8 Hz, H-4) ; 4,25-4,39 (m, 4H, H-2, H-5, H-a) ; 4,10-4,15 (m, 1H, H-6a) ; 3,97-4,03 (m, 1H, H-1a) ; 3,64-4,77 (m, 2, H-1b, H-6b) ; 2,73 (d, 1H, *J* = 8,5 Hz, OH) ; 2.49 (t, 1H, *J* = 2,4 Hz, H-c). RMN ¹³C (CDCl₃, 75 MHz) : δ 81,6 (C-3) ; 80,2 (C-4) ; 80,0 (C-b) ; 78,4 (C-c) ; 75,2 (C-2) ; 74,1 (C-1) ; 72,1 (C-5) ; 70,9 (C-6) ; 57,5 (C-a).

**1,4:3,6-Dianhydro-5-*O*-propargyl-D-sorbitol [8]** : RMN ¹H (CDCl₃, 300 MHz) : δ 4,68 (t, 1H, *J* = 4,3 Hz, H-4) ; 4,38 (d, 1H, *J* = 4,3 Hz, H-3) ; 4,19-4,26 (m, 4H, H2, H-5, H-a) ; 3,88-3,93 (m, 3H, H-1a, H-1b, H-6a) ; 3,53-3,58 (m, 1H, H-6b) ; 2,42 (t, 1H, *J* = 2,4 Hz, H-c) ; 2,31-2,33 (m, 1H, OH). RMN ¹³C (CDCl₃, 75 MHz) : δ 88,3 (C-3) ; 80,0 (C-4) ; 79,2 (C-b) ; 78,7 (C-c) ; 75,7 (C-2, C-5) ; 75,2 (C-1) ; 70,1 (C-6) ; 57,7 (C-a).

**1,4:3,6-Dianhydro-2-*O*-propargyl-D-sorbitol [9] :** RMN ¹H (CDCl₃, 300 MHz) : δ 4,58 (t, 1H, *J* = 4,6 Hz, H-4) ; 4,49 (d, 1H, *J* = 4,6 Hz, H-3) ; 4,19-4,30 (m, 4H, H-2, H5, H-a) ; 3,81-4,07 (m, 3H, H-1a, H-6a, H-6b) ; 3,51-3,56 (m, 1H, H-1b) ; 2,74-2,76 (m, 1H, OH) ; 2,46 (t, 1H, *J* = 2,4 Hz, H-c). RMN ¹³C (CDCl₃, 75 MHz) : δ 85,5 (C-3) ; 83,0 (C-2) ; 81,7 (C-4) ; 78,9 (C-b) ; 75,0 (C-c) ; 73,3 (C-1) ; 72,9 (C-6) ; 72,1 (C-5) ; 56,7 (C-a).

**1,4:3,6-Dianhydro-5-*O*-propargyl-L-iditol [10] :** RMN ¹H (CDCl₃, 300 MHz) : δ 4,72 (d, 1H, *J* = 3,7 Hz, H-4) ; 4,53 (d, 1H, *J* = 3,7 Hz, H-3) ; 4,33-4,35 (m, 1H, H-5) ; 4,21-4,23 (m, 3H, H-2, H-a) ; 3,82-3,94 (m, 4H, H-1a, H-1b, H-6a, H-6b) ; 2,47 (t, 1H, *J* = 2,4 Hz, H-c). RMN ¹³C (CDCl₃, 75 MHz) : δ 87,5 (C-3) ; 84,7 (C-4) ; 82,4 (C-2) ; 80,0 (C-b) ; 75,6 (C-c) ; 75,1 (C-5) ; 74,2-71,8 (C-1, C-6) ; 56,8 (C-a).

### 5. Synthèse des monoazotures :

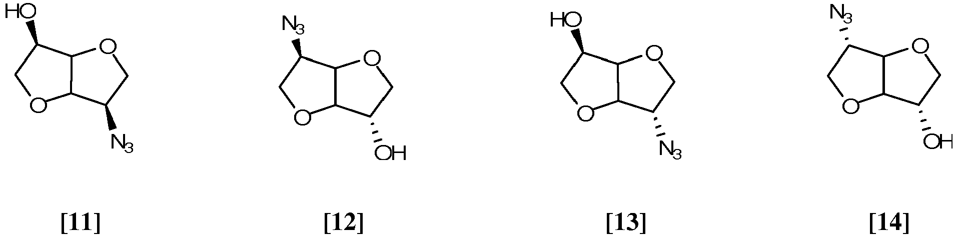

Les monoazotures sont synthétisés par différents procédés :
- procédé décrit par Thiem en utilisant 3 équivalents molaire de groupe partant (tosylate ou triflate) et d'azoture de sodium à la place de 6 équivalents molaire de groupe partant,
- procédé décrit dans l'exemple 7.

### Exemple 7: synthèse du 1,4:3,6-Dianhydro-5-azido-5-deoxy-L-iditol [14]

Sous agitation, sous argon et à température ambiante, de l'isosorbide (fourni par ROQUETTE FRERES) (5,0 g, 34,2 mmol) et de la triphénylphosphine (commercialisé par Sigma-Aldrich^{®} sous le nom triphenylphosphine 98,5 %) (17,9 g, 68,5 mmol) sont dissous dans du diméthylformamide distillé (140 mL) puis une solution d'acide hydrazoïque 5 % dans du toluène (70 mL, 68,5 mmol), fraichement préparée à partir de l'azoture de sodium (commercialisé par Alfa Aesar sous le nom sodium azoture 99 %) est additionnée. Le milieu réactionnel est abaissé à 0 °C et du diisopropylazodicarboxylate (commercialisé par Aldrich^{®} sous le nom diisopropylazodicarboxylate 95 %) (13,6 mL, 68,5 mmol) est additionné. La réaction est poursuivie à température ambiante pendant 2h30. L'excès d'acide hydrazoïque est neutralisé par addition d'une solution d'hydroxyde de sodium 1N (5 mL) puis le diméthylformamide est évaporé sous pression réduite. Le résidu obtenu est repris dans 300 mL d'eau et le milieu réactionnel brut est extrait avec du dichlorométhane (3 x 250 mL). La phase organique est séchée sur sulfate de magnésium, concentrée sous pression réduite et le mélange brut est chromatographié sur gel de silice avec comme éluant le mélange éther de pétrole / acétate d'éthyle (80 / 20).
Le monoazoture **[14]** est obtenu sous forme d'huile avec un rendement de 81 %.

Les monoazotures **[11], [12], [13]** et **[14]** ont également été synthétisés par les procédés ci-dessus décrits. Les résultats obtenus sont récapitulés dans le tableau ci-dessous.

| **Monoazoture** | **[11]** | **[12]** | **[13]** | **[14]** |
|---|---|---|---|---|
| **Réactif inital** | isosorbide | isoidide | isomannide | isosorbide |
| **Procédé** | A | A | A | B |
| **Rendement (%)*** | 1 | 18 | 27 | 81 |

| | | | | |
|---|---|---|---|---|
| A : procédé décrit par Thiem B : procédé décrit dans l'exemple 7 * rendement molaire calculé à partir de l'isosorbide, isomannide ou isoidide. | | | | |

### 6. Synthèse des monomères "AB" :

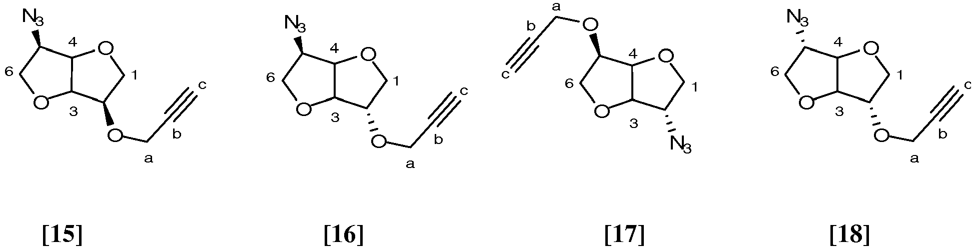

La synthèse des monomères "AB" est réalisée soit à partir des monoalcynes **[7]**, **[8]**, **[9]**, **[10]** (exemple 8) soit à partir des monoazotures **[11]**, **[12]**, **[13]**, **[14]** (exemple 9).

### Exemple 8: synthèse du 1,4:3,6-Dianhydro-5-azido-2-O-propargyl-5-deoxy-D-mannitol [15]

Le monoalcyne **[8]** (200 mg, 1,1 mmol) et de la pyridine (0,4 mL, 5,5 mmol) sont dissous dans du dichlorométhane (5 mL) à température ambiante sous argon. La solution est abaissée à 0 °C puis de l'anhydride triflique (commercialisé par Acros organic sous le nom trifluorométhane sulfonic anhydride 98 %) (0,23 mL, 1,4 mmol) est additionné sous argon. Le milieu réactionnel est maintenu à température ambiante pendant 15 minutes puis il est lavé avec une solution saturée en chlorure d'ammonium (2 x 10 mL). La phase organique est séchée sur sulfate de magnésium, concentrée sous pression réduite. Le triflate intermédiaire brut est dissous dans du diméthylformamide (5 mL) et de l'azoture de sodium (commercialisé par Alfa Aesar sous le nom sodium azoture 99 %) (212 mg, 3,3 mmol) est ajouté. Le milieu réactionnel est maintenu à température ambiante pendant 1h30 puis le diméthylformamide est évaporé sous pression réduite. Le résidu obtenu est repris dans 30 mL d'eau puis est extrait avec du dichlorométhane (2 x 30 mL). La phase organique est séchée sur sulfate de magnésium, concentrée sous pression réduite et le mélange brut est chromatographié sur gel de silice avec comme éluant le mélange heptane / acétate d'éthyle (85 / 15).
Le composé difonctionnel **[15]** est obtenu sous forme d'huile avec un rendement de 45 %.

### Exemple 9 : synthèse du 1,4:3,6-Dianhydro-5-azido-2-O-propargyl-5-deoxy-D-sorbitol [16]

A température ambiante et sous argon, le monoazoture **[12]** (0,85 g, 5 mmol) est dissous dans du diméthylformamide (15 mL). La solution est abaissée à 0 °C puis l'hydrure de sodium à 60 % dans la paraffine (commercialisé par Aldrich^{®} sous le nom sodium hydride 60 % dispersion in mineral oil) (0,30 g, 7,5 mmol) et une quantité catalytique d'éther couronne (18-crown-6, commercialisé par Aldrich^{®} sous le nom 18-crown-6 99 %) sont additionnés. Au retour à température ambiante, du bromure de propargyle à 80 % dans du toluène (commercialisé par Aldrich^{®} sous le nom propargyl bromide 80 % wt in toluene) (0,82 mL, 7,5 mmol) est ajouté. La réaction est poursuivie à température ambiante pendant 12h. L'excès d'hydrure de sodium est neutralisé par addition d'eau (3 mL) puis le diméthylformamide est évaporé sous pression réduite. Le résidu obtenu est repris dans 50 mL d'eau et le dialcyne brut est extrait avec du dichlorométhane (3 x 30 mL). La phase organique est séchée sur sulfate de magnésium, concentrée sous pression réduite et le mélange brut est chromatographié sur gel de silice avec comme éluant le mélange éther de pétrole / acétate d'éthyle (85 / 15).
Le composé **[16]** est obtenu sous la forme d'un solide jaune avec un rendement de 84 %.

Les composés **[15], [16], [17]** et **[18]** ont également été synthétisés par les procédés ci-dessus décrits. Les résultats obtenus sont récapitulés dans le tableau ci-dessous.

| **Composé** | **[15]** | **[16]** | **[17]** | **[18]** |
|---|---|---|---|---|
| **Réactif initial** | [8] | [12] | [13] | [14] |
| **Procédé** | A | B | B | B |
| **Rendement (%)*** | 45 | 84 | 85 | 94 |

| | | | | |
|---|---|---|---|---|
| A : procédé décrit dans l'exemple 8 B : procédé décrit dans l'exemple 9 * rendement molaire calculé à partir de l'isosorbide, isomannide ou isoidide. | | | | |

### Caractérisation des monomètres "AB" :

**1,4:3,6-Dianhydro-5-azido-2-*O*-propargyl-5-deoxy-D-mannitol [15]** : RMN ¹H (C₂D₆SO, 300 MHz) : δ 4,67 (t, 1H, *J* = 4,6 Hz, H-4) ; 4,54 (t, 1H, *J* = 4,6 Hz, H-3) ; 4,14-4,30 (m, 3H, H-2, H-a) ; 3,91-4,01 (m, 3H, H-1a, H-5, H-6a) ; 3,48-3,60 (m, 3H, H-1b, H-6b, H-c). RMN ¹³C (C₂D₆SO, 75 MHz) : δ 82,5 (C-4) ; 80,3 (C-3) ; 80,0 (C-b) ; 78,4 (C-2) ; 77,4 (C-c) ; 69,7-70,3 (C-1, C-6) ; 61,3 (C-5) ; 56,9 (C-a). MSHR: *m*/*z* calculé pour C₉H₁₁N₃O₃ (m/z+H) : 210,0879 ; trouvé : 210,0878.

**1,4:3,6-Dianhydro-5-azido-2-*O*-propargyl-5-deoxy-D-sorbitol** [16] : RMN ¹H (C₂D₆SO, 300 MHz) : δ 4,73 (t, 1H, *J* = 4,6 Hz, H-4) ; 4,49 (d, 1H, *J* = 4,6 Hz, H-3) ; 4,23 (d, 2H, *J =* 2,4 Hz, H-a) ; 4,11-4,12 (m, 1H, H-2) ; 3,94-4,10 (m, 2H, H-1b, H-5) ; 3,79-3,87 (m, 2H, H-1a, H-6a) ; 3,51-3,57 (1H, H-6b) ; 3,49 (t, 1H, *J* = 2,4 Hz, H-c). RMN ¹³C (C₂D₆SO, 75 MHz) : δ 85,5 (C-4) ; 82,4 (C-3) ; 82,3 (C-2) ; 78,9 (C-b) ; 77,3 (C-c) ; 69,0-72,4 (C-1, C-6) ; 61,4 (C-5) ; 56,0 (C-a). MSHR: *m*/*z* calculé pour C₉H₁₁N₃O₃ (m/z+H) : 210,0879 ; trouvé : 210,0880.

**1,4:3,6-Dianhydro-2-azido-5-*O*-propargyl-2-deoxy-D-sorbitol [17] :** RMN ¹H (C₂D₆SO, 300 MHz) : δ 4,64 (t, 1H, *J* = 4,6 Hz, H-4) ; 4,47 (d, 1H, *J* = 4,6 Hz, H-3) ; 4,13-4,31 (m, 4H, H-2, H5, H-a) ; 3,80-4,93 (m, 3H, H-1a, H-1b, H-6a) ; 3,46-3,53 (m, 2H, H-1b, H-c). RMN ¹³C (C₂D₆SO, 75 MHz) : δ 85,8 (C-3) ; 80,0 (C-4, C-b) ; 78,1 (C-5) ; 77,2 (C-c) ; 70,0-72,1 (C-1, C-6) ; 65,5 (C-2) ; 56,9 (C-a). MSHR: *m*/*z* calculé pour C₉H₁₁N₃O₃ (m/z+H) : 210,0879 ; trouvé : 210,0878.

**1,4:3,6-Dianhydro-5-azido-2-*O*-propargyl-5-deoxy-L-iditol [18] :** RMN ¹H (C₂D₆SO, 300 MHz) : δ 4,57 (d, 1H, *J* = 4,1 Hz, H-3) ; 4,50 (d, 1H, *J* = 4,1 Hz, H-4) ; 4,23 (d, 2H, *J* = 2,4 Hz, H-a) ; 4,20-4,21 (m, 1H, H-5) ; 4,09-4,10 (m, 1H, H-2) ; 3,72-3,85 (m, 4H, H-1a, H-1b, H-6a, H-6b) ; 3,49 (t, 1H, *J* = 2,4 Hz, H-c). RMN ¹³C (C₂D₆SO, 75 MHz) : δ 85,1 (C-4) ; 84,6 (C-3) ; 82,8 (C-2) ; 79,9 (C-b) ; 77,4 (C-c) ; 70,8-71,6 (C-1, C-6) ; 65,1 (C-5) ; 56,1 (C-a). MSHR: *m*/*z* calculé pour C₉H₁₁N₃O₃ (m/z+H) : 210,0879 ; trouvé : 210,0880.

### 7. Polyaddition par click-chemistry en solution des monomères "AB"

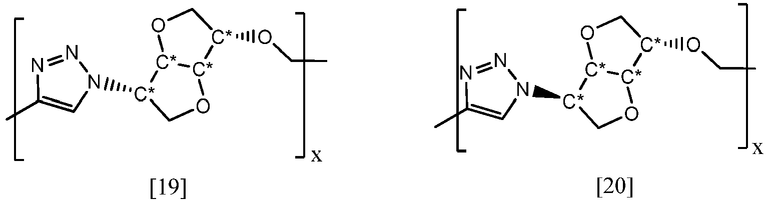

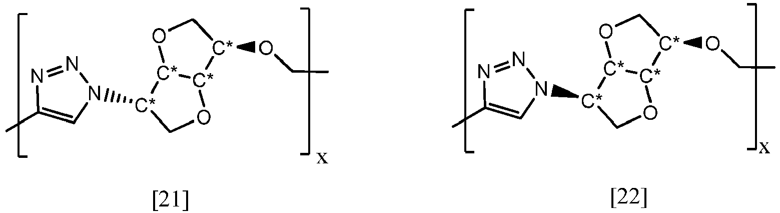

Les polymères **[19]** à **[22]** sont synthétisés par polyaddition par click chemistry selon le procédé décrit dans l'exemple 10.

### Exemple 10 : Polymérisation du monomère "AB" [16]

Le composé **[16]** (98 mg, 0,5 mmol) est dissous dans le diméthylsulfoxide (0,47 mL) à température ambiante. De la triéthylamine (commercialisée par Fluka^{®} sous le nom triethylamine 98 %) (0,09 mL, 0,7 mmol) et un catalyseur CuI.P(OEt)₃ (synthétisé selon le procédé décrit dans Org. Lett., 2006, 8, (17), pp 3761-3764) (1,7 mg, 0,005 mmol) sont additionnés. Le milieu réactionnel est porté à 60 °C pendant 15 heures. Au gel obtenu, 1 mL de diméthylsulfoxide est additionné puis la solution visqueuse formée est précipitée dans l'éthanol (20 mL). Après filtration sur fritté, le précipité isolé est lavé à l'eau (4 x 10 mL), à l'acétone (2 x 10 mL) puis séché à la pompe à vide.
Le polymère **[20]** est obtenu est un solide orange et le rendement massique est de 95 %.

Les polymères **[19], [20], [21]** et **[22]** ont également été synthétisés par les procédés ci-dessus décrits. Les résultats obtenus sont récapitulés dans le tableau ci-dessous.

| **Polymère** | **[19]** | **[20]** | **[21]** | **[22]** |
|---|---|---|---|---|
| **Monomère initial** | [18] | [16] | [17] | [15] |
| **Rendement massique (%)*** | 85 | 95 | 94 | 88 |
| **T_{g} (°C)** | 140 | 146 | 141 | 173 |
| **T_{D10} (°C)**** | 319 | 322 | 309 | 324 |
| **DPn** | 33 | Non déterminé | 36 | Non déterminé |

| | | | | |
|---|---|---|---|---|
| * rendement massique = masse monomère inital / masse polymère purifié et récupéré. ** température à laquelle 10 % de perte de masse du polymère. | | | | |

### Caractérisation des polymères "AB" :

**Polymère [19] :** RMN ¹H (C₂D₆SO, 300 MHz) : δ 8,16 (s, 1H, H-a) ; 5,19-5,21 (m, 1H, H-5); 4,77-4,84 (m, 2H, H-3, H-4) ; 4,60-4,62 (m, 2H, H-b) ; 4,07-4,17 (m, 3H, H-2, H-6a, H-6b) ; 3,88-3,90 (m, 2H, H-1a, H-1b).
**Polymère [20] :** RMN ¹H (C₂D₆SO, 300 MHz) : δ 8,18 (s, 1H, H-a) ; 5,26-5,33 (m, 1H, H-5); 4,78-4,81 (m, 1H, H-4) ; 4,70-4,71 (m, 1H, H-3) ; 4,59-4,61 (m, 2H, H-b) ; 3,89-4,30 (m, 5H, H-1a, H-1b, H-2, H-6a, H-6b).
**Polymère [21] :** RMN ¹H (C₂D₆SO, 300 MHz) : δ 8,15 (s, 1H, H-a) ; 5,20-5,22 (m, 1H, H-5); 4,58-4,82 (m, 4H, H-3, H-4, H-b) ; 3,59-4,17 (m, 5H, H-1a, H-1b, H-2, H-6a, H-6b).
**Polymère [22] :** RMN ¹H (C₂D₆SO, 300 MHz) : δ 8,17 (s, 1H, H-a) ; 5,27-5,34 (m, 1H, H-5); 4,56-4,81 (m, 4H, H-3, H-4, H-b) ; 4,30-4,36 (m, 1H, H-2) ; 3,54-4,23 (m, 4H, H-1a, H-1b, H-6a, H-6b).

### 8. Polyaddition par click-chemistry en masse des monomères "AB"

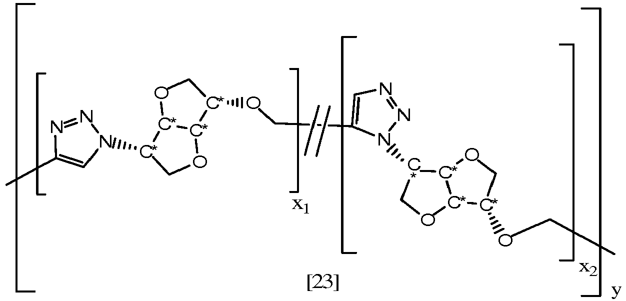

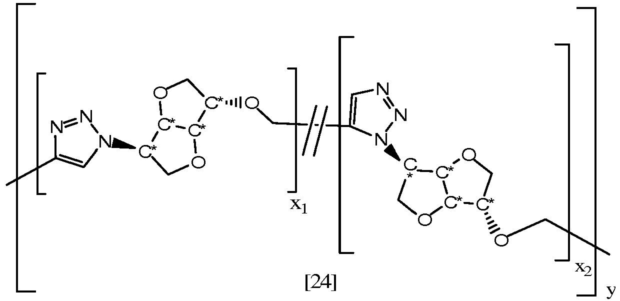

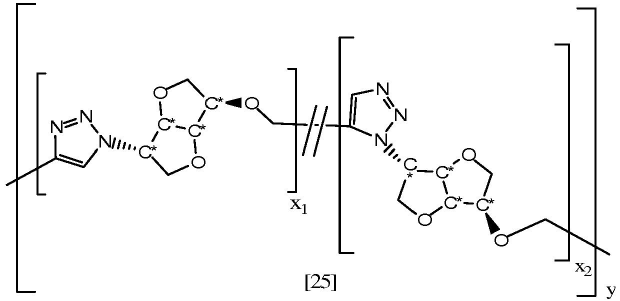

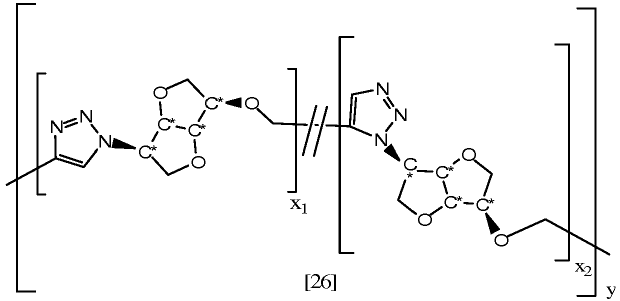

Les polymères **[23]** à **[26]** sont synthétisés en masse sans base ni catalyseur selon le procédé décrit dans l'exemple 11 ou 12.

### Exemple 11 : Polymérisation du monomère "AB" [16]

Le composé **[16]** (5 mg) est introduit dans une capsule DSC. Le polymère **[24]** est alors obtenu sous la forme d'un solide marron après un chauffage de - 90 °C à 250 °C à une vitesse de 20 °C/min en DSC.

### Exemple 12 : Polymérisation du monomère "AB" [18]

Le composé **[18]** (200 mg) disposé entre 2 feuilles de téflon est chauffé sous presse 30 secondes à 200 °C. Le polymère **[23]** est alors obtenu sous la forme d'un film translucide.

Les polymères **[23], [24], [25]** et **[26]** ont également été synthétisés par les procédés ci-dessus décrits. Les résultats obtenus sont récapitulés dans le tableau ci-dessous.

| **Polymère** | **[23]** | **[24]** | **[25]** | **[26]** |
|---|---|---|---|---|
| **Monomère initial** | [18] | [16] | **[17]** | **[15]** |
| **Procédé** | A et B | A | A | A |
| **T_{g} (°C)** | 132 et 137 | 136 | 124 | 159 |
| **DPn** | 55 | 43 | 153 | 47 |
| **x₁/x₂** | 2/1 | 1,9/1 | 1,8/1 | 1,8 / 1 |

| | | | | |
|---|---|---|---|---|
| A : procédé décrit dans l'exemple 11 B : procédé décrit dans l'exemple 12 | | | | |

### Caractérisation des polymères "AB" :

**Polymère [23] :** RMN ¹H (C₂D₆SO, 300 MHz) : δ 8,16-8,17 (m, 0,6 H, H-a) ; 7,76-7,77 (m, 1H, H-a') ; 5,13-5,21 (m, 1H, H-5); 4,62-4,89 (m, 4H, H-3, H-4, H-b) ; 3,89-4,17 (m, 5H, H-1a, H-1b, H-2, H-6a, H-6b).
**Polymère [24] :** RMN ¹H (C₂D₆SO, 300 MHz) : δ 8,16-8,18 (m, 0,6 H, H-a) ; 7,73-7,75 (m, 0,4 H, H-a') ; 5,08-5,31 (m, 1H, H-5); 4,51-4,90 (m, 4H, H-3, H-4, H-b) ; 3,65-4,30 (m, 5H, H-1a, H-1b, H-2, H-6a, H-6b).
**Polymère [25] :** RMN ¹H (C₂D₆SO, 300 MHz) : δ 8,15 (s, 0,6 H, H-a) ; 7,79 (s, 0,4 H, Ha') ; 5,22-5,26 (m, 1H, H-5); 4,58-4,90 (m, 4H, H-3, H-4, H-b) ; 3,59-4,22 (m, 5H, H-1a, H-1b, H-2, H-6a, H-6b).
**Polymère [26] :** RMN ¹H (C₂D₆SO, 300 MHz) : δ 8,18 (s, 0,6 H, H-a) ; 7,77-7,79 (m, 0,4 H, H-a') ; 5,18-5,34 (m, 1H, H-5); 4,57-4,92 (m, 4H, H-3, H-4, H-b) ; 4,30-4,39 (m, 1H, H-2) ; 3,41-4,24 (m, 4H, H-1a, H-1b, H-6a, H-6b).

### 9. Polyaddition par click-chemistry en solution des monomères "AA/BB"

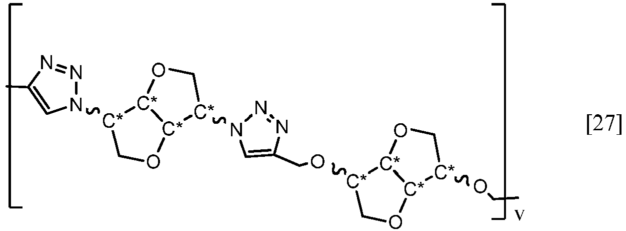

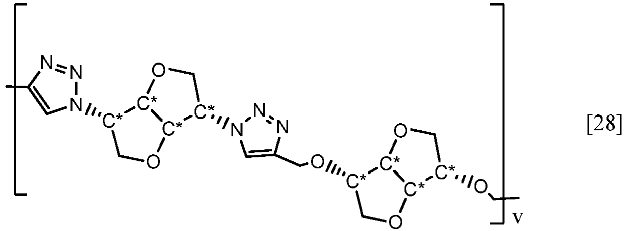

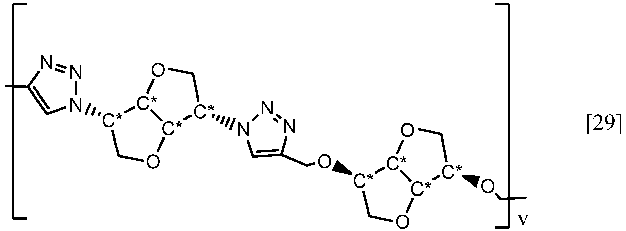

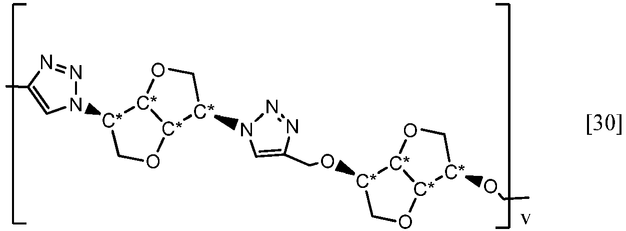

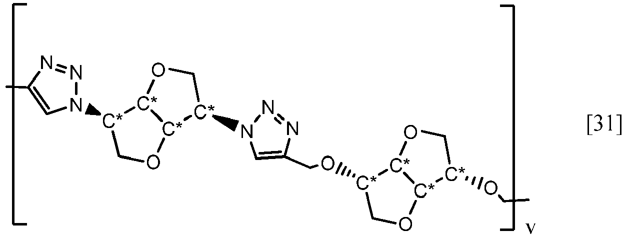

Les polymères **[27]** à **[31]** sont synthétisés par polyaddition par click chemistry selon le procédé décrit dans l'exemple 13.

### Exemple 13 : Polymérisation des monomères "AA" [3] et "BB" [6]

Le diazoture **[3]** (100 mg, 0,5 mmol) et le dialcyne **[6]** (103 mg, 0,5 mmol) sont dissous dans du diméthylsulfoxide (0,94 mL) à température ambiante. De la triéthylamine (commercialisée par Fluka^{®} sous le nom triethylamine 99 %) (0,36 mL, 1,5 mmol) et du catalyseur CuI.P(OEt)₃ (synthétisé selon le procédé décrit dans Org. Lett., 2006, 8, (17), pp 3761-3764) (2,4 mg, 0,005 mmol) sont additionnés. Le milieu réactionnel est porté à 60 °C pendant 15 heures. La solution visqueuse obtenue en fin de réaction est précipitée dans l'éthanol (20 mL). Après filtration sur fritté, le précipité isolé est lavé à l'eau (4 x 10 mL), à l'acétone (2 x 10 mL) puis séché à la pompe à vide.
Le polymère **[28]** obtenu est un solide beige et le rendement massique est de 96 %.

Les polymères **[27], [28], [29], [30]** et **[31]** ont également été synthétisés par le procédé ci-dessus décrit. Les résultats obtenus sont récapitulés dans le tableau ci-dessous.

| **Polymère** | **[27]** | **[28]** | **[29]** | **[30]** | **[31]** |
|---|---|---|---|---|---|
| **Diazoture initial** | [2] | [3] | [3] | [1] | [1] |
| **Dialcyne initial** | [5] | [6] | [4] | [4] | [6] |
| **Rendement** massique **(%)*** | 78 | 96 | 93 | 83 | 85 |
| **T_{g} (°C)** | 126 | 125 | 150 | 166 | 151 |
| **T_{D10} (°C)**** | 321 | 318 | 311 | 308 | 327 |
| **DPn** | 28 | 39 | 40 | 21 | 21 |

| | | | | | |
|---|---|---|---|---|---|
| * rendement massique = (masse diazoture + masse dialcyne) / masse polymère purifié et récupéré. ** température à laquelle 10 % de perte de masse du polymère. | | | | | |

### Caractérisation des polymères "AA/BB" :

**Polymère [27] :** RMN ¹H (C₂D₆SO, 300 MHz) : δ 8,26 (s, 1H, H-a) ; 8,17 (s, 1H, H-a) ; 5,37-5,39 (m, 2H, H-2, H-5) ; 5,03-5,10 (m, 2H, H-3, H-4) ; 4,48-4,74 (m, 6H, H-3', H-4', H-b) ; 3,70-4,49 (m, 9H, H-1a, H-1b, H-6a, H-6b, H-1a', H-1b', H-2', H-5', H-6a') ; 3,39-3,44 (m, 1H, H-6b').
**Polymère [28] :** RMN ¹H (C₂D₆SO, 300 MHz) : δ 8,18 (s, 2H, H-a) ; 5,37-5,39 (m, 2H, H-2, H-5) ; 5,07-5,13 (m, 2H, H-3, H-4) ; 3,70-4,59 (m, 16H, H-1a, H-1b, H-6a, H-6b, H-1a', H-1b', H-2', H-3', H-4',H-5', H-6a', H-6b', H-b).
**Polymère [29] :** RMN ¹H (C₂D₆SO, 300 MHz) : δ 8,19 (s, 2H, H-a) ; 5,36-5,38 (m, 2H, H-2, H-5) ; 5,12-5,14 (m, 2H, H-3, H-4) ; 4,52-4,68 (m, 6H, H-3', H-4', H-b) ; 4,31-4,36 (m, 2H, H-2', H-5') ; 3,41-4,17 (m, 8H, H-1a, H-1b, H-6a, H-6b, H-1a', H-1b', H-6a', H-6b').
**Polymère [30]** : RMN ¹H (C₂D₆SO, 300 MHz) : δ 8,25 (s, 2H, H-a) ; 5,43-5,48 (m, 2H, H-2, H-5) ; 4,96-4,98 (m, 2H, H-3, H-4) ; 4,56-4,76 (m, 6H, H-3', H-4', H-b) ; 4,30-4,33 (m, 2H, H-2', H-5') ; 3,43-4,14 (m, 8H, H-1a, H-1b, H-6a, H-6b, H-1a', H-1b', H-6a', H-6b').
**Polymère [31] :** RMN ¹H (C₂D₆SO, 300 MHz) : δ 8,25 (s, 2H, H-a) ; 5,40-5,47 (m, 2H, H-2, H-5) ; 4,96-4,97 (m, 2H, H-3, H-4) ; 4,53-4,62 (m, 6H, H-3', H-4', H-b) ; 4,26-4,32 (m, 2H, H-2', H-5') ; 3,70-4,07 (m, 8H, H-1a, H-1b, H-6a, H-6b, H-1a', H-1b', H-6a', H-6b').

## Revendications

1. Polymères obtenus à partir de monomère(s) dérivé(s) de 1,4 : 3,6-dianhydrohexitol de formule I 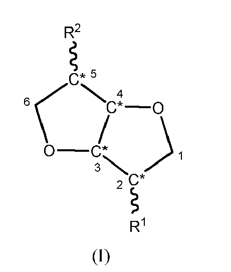 dans laquelle :
- R¹ est un radical : 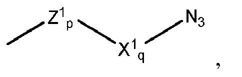
- Z²R³,ou
- Z¹H ;
avec p = 0 ou 1 et q = 0 ou 1 ;
- R² est un radical -Z²R³, les radicaux -Z²R³ étant identiques ou différents quand R¹ et R² correspondent chacun à -Z²R³ ;
- Z¹, Z² représentent indépendamment un radical divalent correspondant à : 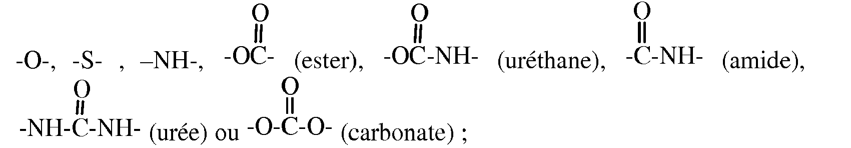
- R³ est un radical divalent de formule : -X²-C≡Y;
- -X¹-, X²- sont indépendamment des liaisons covalentes ou des rotules divalentes formées par un groupement (cyclo)aliphatique et/ou aromatique, halogénés ou non et contenant ou non des hétéroatomes, de préférence un groupement -(CH₂)ₙ- avec n compris entre 1 et 100 ;
- Y représente CH ou N ;
lesdits polymères étant
- soit des polymères de type AB **caractérisés en ce qu'**ils répondent à la formule VI suivante : 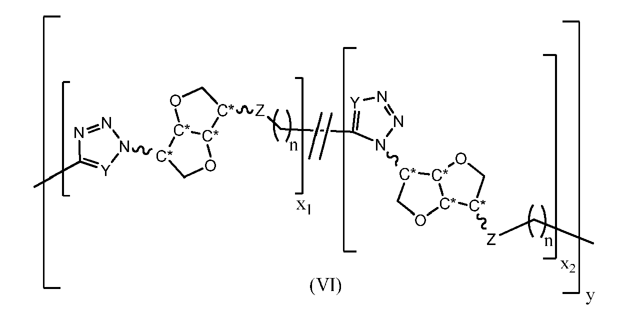 dans laquelle
- y est le degré de polymérisation moyen,
- x₁ et x₂ varient de 0 à 100% en fonction de la présence (ou non) et de la nature du catalyseur,
- n est compris entre 1 et 100,
- Y est CH ou N,
- Z est O, S ou NH ;
- soit des polymères de type AA/BB **caractérisés en ce qu'**ils répondent à la formule VII suivante : 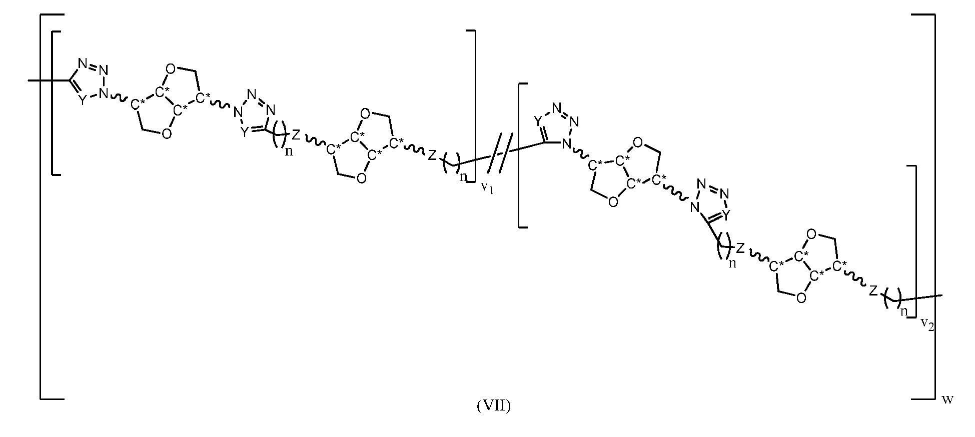 dans laquelle
- w est le degré de polymérisation moyen,
- v₁ et V₂ varient de 0 à 100% en fonction de la présence (ou non) et de la nature du catalyseur,
- n est compris entre 1 et 100,
- Y est CH ou N,
- Z est O, S ou NH.

2. Polymères selon la revendication 1, **caractérisés en ce qu'**ils sont constitués à 20 % massique, de préférence à 40 % massique, et encore plus préférentiellement de 60 % massique, de motif 1,4 :3,6-dianhydrohexitol d'origine naturelle.

3. Procédé de préparation des polymères de type AB ou des polymères de type AA/BB tels que définis à la revendication 1 ou 2, **caractérisé en ce qu'**il comprend une cycloaddition 1,3 dipolaire.

4. Procédé de préparation selon la revendication 3, **caractérisé en ce que** la réaction de cycloaddition 1,3 dipolaire est une réaction de polymérisation en masse contrôlée par la température et la pression, en présence ou en absence de catalyseur.

5. Procédé de mise en forme d'un matériau polymère notamment par extrusion, d'extrusion-soufflage, ou de moulage par injection ou par compression, **caractérisé en ce que** le matériau polymère mis en oeuvre est choisi parmi les polymères selon l'une ou l'autre des revendications 1 et 2 ou dans lequel le matériau polymère est obtenu, au moins en partie lors de la mise en forme, par polymérisation des monomères tels que définis dans la revendication 1.

6. Procédé de revêtement d'un support par un polymère selon l'une ou l'autre des revendications 1 et 2.

7. Monomère dérivé de 1,4 : 3,6-dianhydrohexitol de formule Ia : 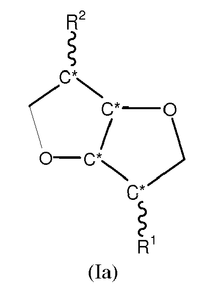 dans laquelle :
- R¹ est un radical : 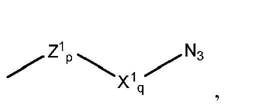
- Z²R³,ou
- Z¹H ;
avec p = 0 ou 1 et q = 0 ou 1,
- R² est un radical -Z²R³, les radicaux -Z²R³ étant identiques ou différents quand R¹ et R² correspondent chacun à -Z²R³;
- Z¹, Z² représentent indépendamment un radical divalent correspondant à : 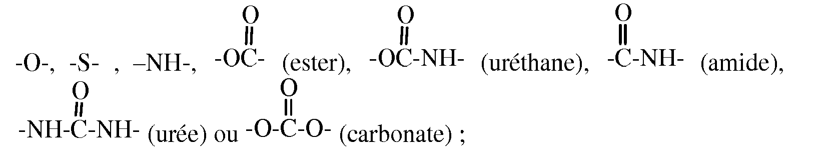
- R³ est un radical divalent de formule : -X²-C≡Y;
- -X¹-, X²- sont indépendamment un groupement -(CH₂)ₙ- avec n compris entre 1 et 100,
- Y représente CH.

8. Monomère selon la revendication 7, **caractérisé en ce qu'**il est de type AA et répond à la formule Ia dans laquelle R¹ et R² sont identiques et représentent un radical -Z²R³ avec :
- Z² représentant un radical divalent correspondant à : 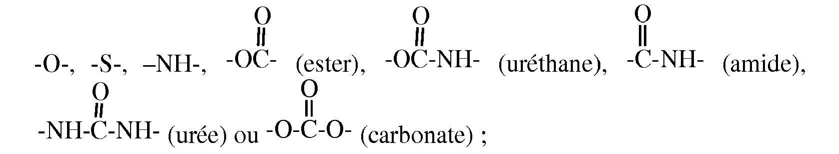
- R³ étant un radical divalent de formule : -X²-C≡Y;
- -X²- étant une liaison covalente ou une rotule divalente formée par un groupement : -(CH₂)ₙ- avec n compris entre 1 et 100, et
- Y représentant CH.

9. Monomère selon la revendication 7, **caractérisé en ce qu'**il est de type AB et répond à la formule Ia dans laquelle
R¹ représentant un radical 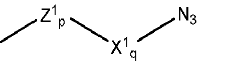 et R² représentant un radical -Z²R³
avec :
- p = 0 ou 1,
- q = 0 ou 1,
- Z¹, Z² représentant un radical divalent correspondant à : 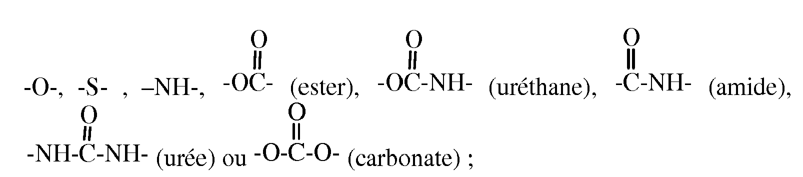
- R³ étant un radical divalent de formule : -X²-C≡Y;
- -X¹-, X²- étant indépendamment des liaisons covalentes ou des rotules divalentes formées par un groupement (cyclo)aliphatique et/ou aromatique, halogénés ou non et contenant ou non des hétéroatomes, de préférence un groupement -(CH₂)ₙ- avec n compris entre 1 et 100 ;
- Y représentant CH ou N.

10. Monomère selon l'une quelconque des revendications 7 à 9, **caractérisé en ce que** :
• -X¹- est une liaison covalente,
• - X²- est un -(CH₂)ₙ- avec 1 ≤ n ≤ 10, de préférence n = 1,
• Z¹ et Z² sont des radicaux -O-, et
• Y est CH.

## Patentansprüche

1. Polymere, gewonnen aus Monomer(en), abgeleitet aus 1,4:3,6-Dianhydrohexitol der Formel I: 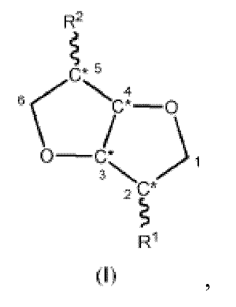 in der:
- R¹ ein Radikal: 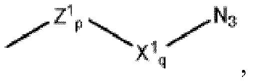
- Z²R³ oder -Z¹H ist;
mit p = 0 oder 1 und q = 0 oder 1;
- R² ein Radikal -Z²R³ ist, wobei die Radikale -Z²R³ identisch oder verschieden sind, wenn R¹ und R² jeweils -Z²R³ entsprechen;
- Z¹, Z² unabhängig ein divalentes Radikal repräsentieren, das Folgendem entspricht: 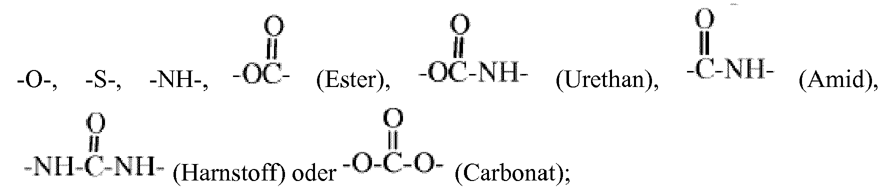
- R³ ein divalentes Radikal der Formel: -X²-C≡Y ist;
- -X¹-, -X²- unabhängig kovalente Bindungen oder divalente Gelenke sind, die durch eine (cyclo)aliphatische und/oder aromatische Gruppierung, die halogeniert oder nicht halogeniert ist und Heteroatome enthält oder nicht enthält, vorzugsweise eine Gruppierung -(CH₂)ₙ-, mit n zwischen 1 und 100, gebildet werden;
- Y CH oder N repräsentiert;
wobei die besagten Polymere
- entweder Polymere vom Typ AB sind, **dadurch gekennzeichnet, dass** sie der folgenden Formel VI entsprechen: 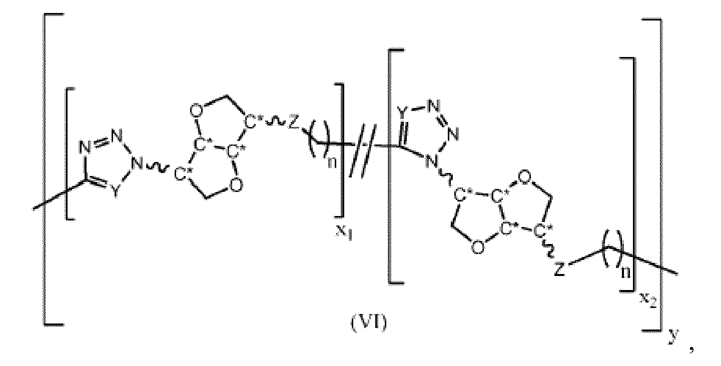 in der
- y der mittlere Polymerisationsgrad ist,
- x₁ und x₂ abhängig vom Vorhandensein (oder Nichtvorhandensein) und von der Art des Katalysators zwischen 0 und 100 % variieren,
- n zwischen 1 und 100 liegt,
- Y CH oder N ist,
- Z O, S oder NH ist;
- oder Polymere vom Typ AA/BB sind, **dadurch gekennzeichnet, dass** sie der folgenden Formel VII entsprechen: 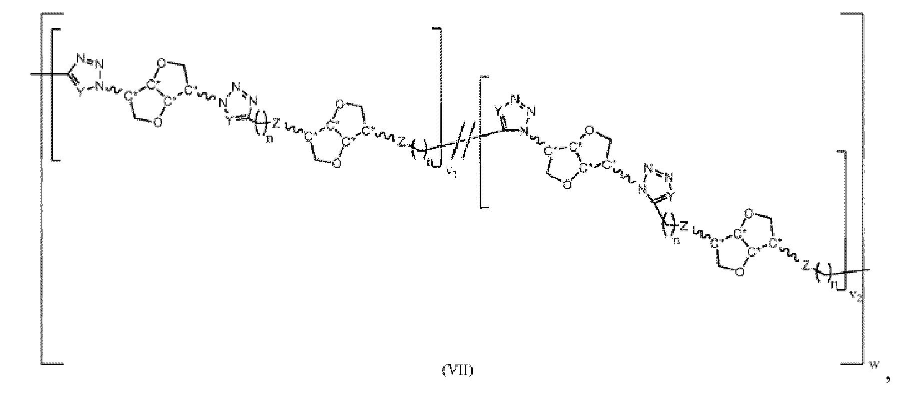 in der
- w der mittlere Polymerisationsgrad ist,
- v₁ und v₂ abhängig vom Vorhandensein (oder Nichtvorhandensein) und von der Art des Katalysators zwischen 0 und 100 % variieren,
- n zwischen 1 und 100 liegt,
- Y CH oder N ist,
- Z O, S oder NH ist.

2. Polymere gemäß Anspruch 1, **dadurch gekennzeichnet, dass** sie zu 20 Massen-%, vorzugsweise zu 40 Massen-% und besonders bevorzugt zu 60 Massen-% aus dem Motiv 1,4:3,6-Dianhydrohexitol natürlichen Ursprungs bestehen.

3. Verfahren zur Herstellung von Polymeren vom Typ AB oder von Polymeren vom Typ AA/BB wie in Anspruch 1 oder 2 definiert, **dadurch gekennzeichnet, dass** es eine 1,3-dipolare Cycloaddition umfasst.

4. Herstellungsverfahren gemäß Anspruch 3, **dadurch gekennzeichnet, dass** die 1,3-dipolaren Cycloadditionsreaktion eine durch die Temperatur und den Druck kontrollierte Blockpolymerisationsreaktion mit vorhandenem oder nicht vorhandenem Katalysator ist.

5. Verfahren zum Formen eines Polymermaterials, insbesondere durch Extrudieren, Blasextrudieren oder Spritz- oder Druckgießen, **dadurch gekennzeichnet, dass** das umgesetzte Polymermaterial aus den Polymeren gemäß einem der Ansprüche 1 und 2 ausgewählt wird, oder in dem das Polymermaterial, zumindest teilweise beim Formen, durch Polymerisation von Monomeren wie in Anspruch 1 definiert gewonnen wird.

6. Verfahren zur Beschichtung eines Trägers durch ein Polymer gemäß einem der Ansprüche 1 und 2.

7. Monomer, abgeleitet aus 1,4:3,6-Dianhydrohexitol der Formel Ia: 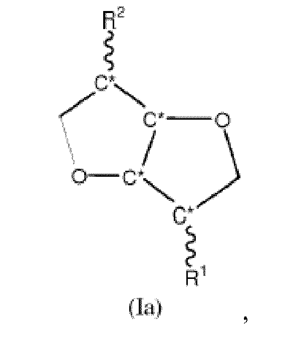 in der:
- R¹ ein Radikal: 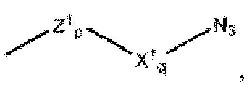 -Z²R³ oder -Z¹H ist; mit p = 0 oder 1 und q = 0 oder 1,
- R² ein Radikal -Z²R³ ist, wobei die Radikale -Z²R³ identisch oder verschieden sind, wenn R¹ und R² jeweils -Z²R³ entsprechen;
- Z¹, Z² unabhängig ein divalentes Radikal repräsentieren, das Folgendem entspricht: 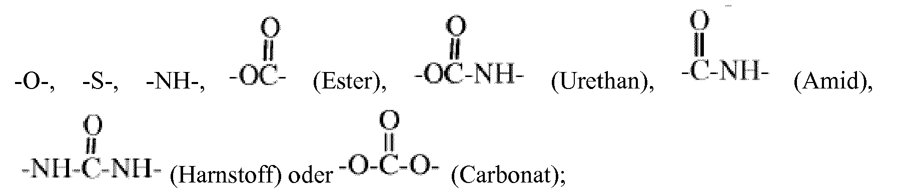
- R³ ein divalentes Radikal der Formel: -X²-C≡Y ist;
- -X¹-, -X²- unabhängig eine Gruppierung -(CH₂)ₙ- sind, mit n zwischen 1 und 100,
- Y CH repräsentiert.

8. Monomer gemäß Anspruch 7, **dadurch gekennzeichnet, dass** es vom Typ AA ist und der Formel Ia entspricht, in der R¹ und R² identisch sind und ein Radikal -Z²R³ repräsentieren, wobei:
- Z² ein divalentes Radikal repräsentiert, das Folgendem entspricht: 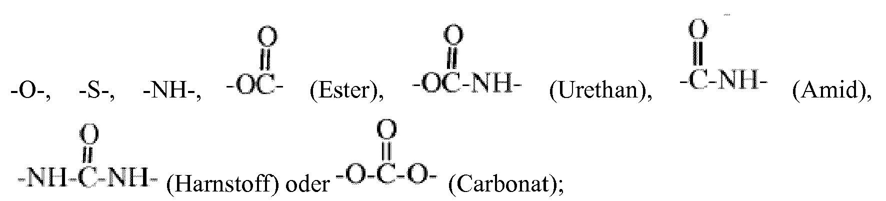
- R³ ein divalentes Radikal der Formel: -X²-C≡Y ist;
- -X²- eine kovalente Bindung oder ein divalentes Gelenk ist, gebildet durch eine Gruppierung: -(CH₂)ₙ-, mit n zwischen 1 und 100, und
- Y CH repräsentiert.

9. Monomer gemäß Anspruch 7, **dadurch gekennzeichnet, dass** es vom Typ AB ist und der Formel Ia entspricht, in der:
R¹ ein Radikal 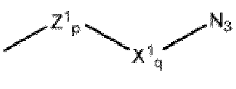 repräsentiert und R² ein Radikal -Z²R³ repräsentiert,
wobei:
- p = 0 oder 1,
- q = 0 oder 1,
- Z¹, Z² ein divalentes Radikal repräsentieren, das Folgendem entspricht: 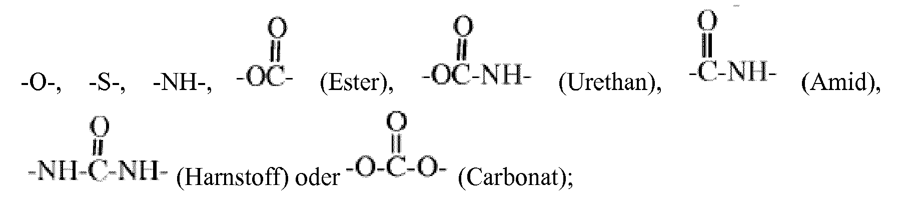
- R³ ein divalentes Radikal der Formel: -X²-C=Y ist;
- -X¹-, -X²- unabhängig kovalente Bindungen oder divalente Gelenke sind, die durch eine (cyclo)aliphatische und/oder aromatische Gruppierung, die halogeniert oder nicht halogeniert ist und Heteroatome enthält oder nicht enthält, vorzugsweise eine Gruppierung -(CH₂)ₙ-, mit n zwischen 1 und 100, gebildet werden;
- Y CH oder N repräsentiert.

10. Monomer gemäß einem der Ansprüche 7 bis 9, **dadurch gekennzeichnet, dass**:
- -X¹- eine kovalente Bindung ist,
- -X²- ein -(CH2)ₙ ist, mit 1 ≤ n ≤ 10, vorzugsweise n = 1,
- Z¹ und Z² Radikale -O- sind und
- Y CH ist.

## Claims

1. Polymers obtained from:
* monomer(s) derived from 1,4:3,6-dianhydrohexitol of formula I 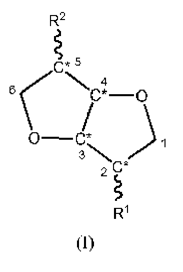 wherein:
- R¹ is a radical: 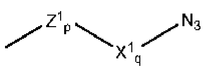 -Z²R³, or
-Z¹H;
with p = 0 or 1 and q = 0 or 1;
- R² is a -Z²R³ radical, the -Z²R³ radicals being identical or different when R¹ and R² each correspond to -Z²R³;
- Z¹, Z² represent independently a divalent radical corresponding to: 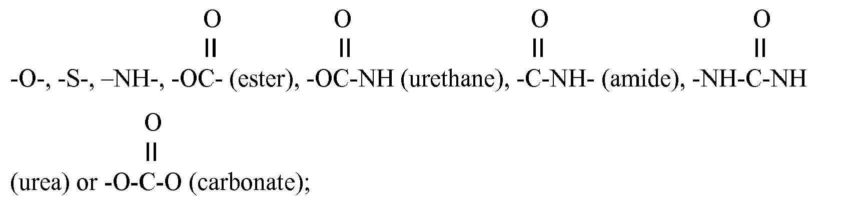
- R³ is a divalent radical of formula: -X²-C≡Y;
- -X¹-, -X²- are independently covalent bonds or divalent balls formed by a (cyclo)aliphatic and/or aromatic group, either halogenated or not and containing or not containing heteroatoms, preferably a -(CH₂)ₙ- group with n being between 1 and 100;
- Y represents CH or N;
said polymers being
- either polymers of the AB type, **characterised in that** they respond to the following formula VI: 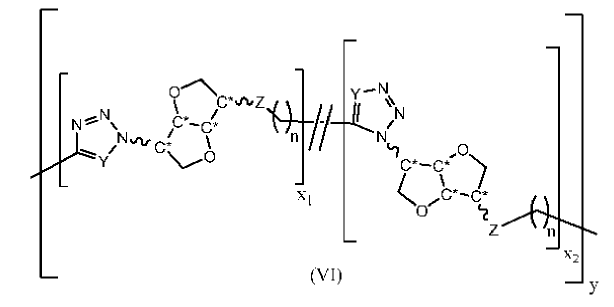
wherein
- y is the average degree of polymerisation,
- x₁ and x₂ vary from 0 to 100% as a function of the presence (or not) of a catalyst and the type of catalyst,
- n is between 1 and 100,
- Y is CH or N,
- Z is O, S or NH.
- or polymers of the AA/BB type, **characterised in that** they respond to the following formula VII: 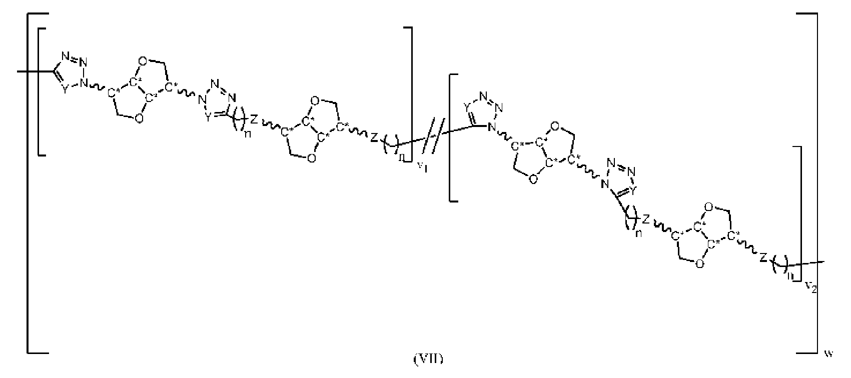 wherein
- w is the average degree of polymerisation,
- v₁ and v₂ vary from 0 to 100% as a function of the presence (or not) of a catalyst and the type of catalyst,
- n is between 1 and 100,
- Y is CH or N,
- Z is O, S or NH.

2. Polymers according to claim 1, **characterised in that** they consist of 20% by mass, preferably 40% by mass, and even more preferably 60% by mass of the motif 1,4:3,6-dianhydrohexitol of natural origin.

3. Method for the preparation of AB type polymers or AA/BB type polymers as defined in claim 1 or 2, **characterised in that** it comprises a 1,3-dipolar cycloaddition.

4. Method of preparation according to claim 3, **characterised in that** the reaction of 1,3 dipolar cycloaddition is a mass polymerisation reaction controlled by temperature and pressure, in the presence or absence of a catalyst.

5. Method for shaping a polymer material in particular by extrusion, blowing extrusion or injection moulding or compression moulding, **characterised in that** the polymer material used is selected from polymers according to any one of claims 1 to 2 or wherein the polymer material is obtained at least partly during the shaping, by polymerisation of monomers as defined in claim 1.

6. Method for coating a support with a polymer according to any one of claims 1 to 2.

7. Monomer derived from 1,4:3,6-dianhydrohexitol of formula Ia: 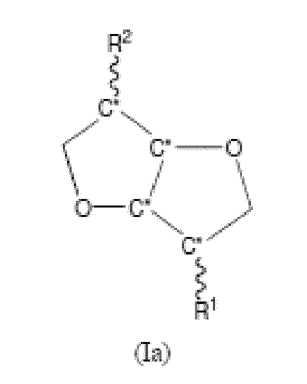 wherein:
- R¹ is a radical: 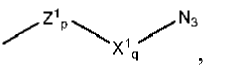 -Z²R³ or
-Z¹H;
with p = 0 or 1 and q = 0 or 1,
- R² is a -Z²R³ radical, the -Z²R³ radicals being identical or different when R¹ and R² each correspond to -Z²R³;
- Z¹, Z² represent independently a divalent radical corresponding to: 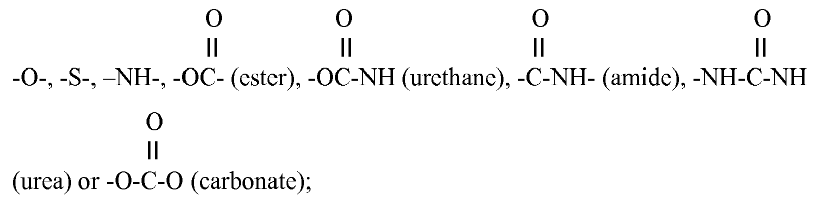
- R³ is a divalent radical of formula: -X²-C≡Y;
- -X¹-, -X²- are independently a -(CH₂)ₙ- group with n being between 1 and 100,
- Y represents CH.

8. Monomer according to claim 7, **characterised in that** it is of the AA type and responds to the formula Ia wherein R¹ and R² are identical and represent a -Z²R³ radical with:
- Z² representing a divalent radical corresponding to: 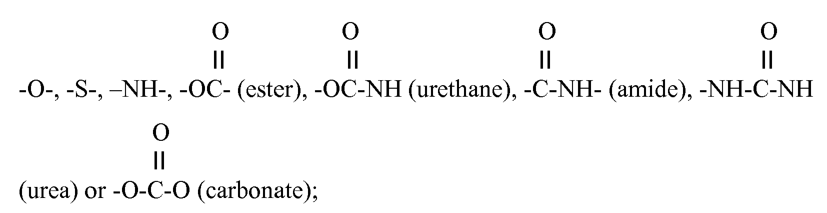
- R³ being a divalent radical of formula: -X²-C≡Y;
- -X²- being a covalent bond or a divalent ball formed by a -(CH₂)ₙ group with n being between 1 and 100, and
- Y representing CH.

9. Monomer according to claim 7, **characterised in that** it is of the AB type and responds to the formula Ia wherein
R¹ represents a radical 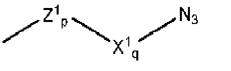 and R² represents a -Z²R³ radical with:
- p = 0 or 1,
- q = 0 or 1,
- Z¹, Z² representing a divalent radical corresponding to: 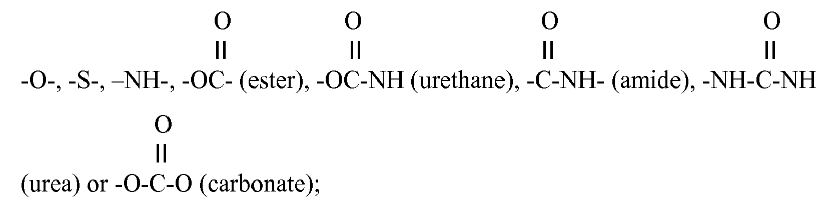
- R³ being a divalent radical of formula: -X²-C≡Y;
- -X¹-, -X²- being independently covalent bonds or divalent balls formed by a (cyclo)aliphatic and/or aromatic group, either halogenated or not halogenated and either containing heteroatoms or not, preferably a -(CH₂)ₙ group with n being between 1 and 100;
- Y representing CH or N.

10. Monomer according to any one of claims 7 to 9, **characterised in that**;
- -X¹- is a covalent bond,
- -X²- is a -(CH₂)ₙ- with 1≤n≤10, preferably n=1,
- Z¹ and Z² are -O- radicals and
- Y is CH.
